# EUROPEAN PATENT SPECIFICATION

(11) **EP 0 690 916 B1**
(45) Date of publication and mention of the grant of the patent: **02.03.2005**
(21) Application number: 94911683.4
(22) Date of filing: 23.03.1994
(51) Int. Cl.: C12N 15/31, C12N 15/32, C12N 15/82, A01H 5/00, A01N 63/00, C12P 1/04, C12N 1/21, C12Q 1/68, C12P 21/08

(54) **PESTICIDAL PROTEINS AND STRAINS**
PESTIZID-PROTEINE UND STÄMME
SOUCHES ET PROTEINES PESTICIDES

(30) Priority: 25.03.1993 US 37057
(43) Date of publication of application: 10.01.1996
(60) Divisional application: 04015832.1
(73) Proprietor: Syngenta Participations AG, 4058 Basel (CH)
(72) Inventor: WARREN, Gregory W., Cary, NC 27513 (US); KOZIEL, Michael G., Cary, NC 27511 (US); MULLINS, Martha A., Youngsville, NC 27596 (US); NYE, Gordon J., Apex, NC 27502 (US); DESAI, Nalini, Cary, NC 27511 (US); CARR, Brian, Raleigh, NC 27606 (US); KOSTICHKA, N. Kristy, Durham, NC 27713 (US)
(86) International application number: PCT/US1994/003131
(87) International publication number: WO 1994/021795

(56) References cited:
- WO-A-88/08880
- WO-A-90/13651
- WO-A-91/16432
- WO-A-91/16434
- CURR MICROBIOL 17 (6). 1988. 347-350 SEKAR, V. 'THE INSECTICIDAL CRYSTAL PROTEIN GENE IS EXPRESSED IN VEGETATIVE CELLS OF BACILLUS -THURINGIENSIS-VAR- TENEBRIONIS.'
- BIOTECHNOLOGY vol. 11 , February 1993 , NEW YORK US pages 194 - 200 KOZIEL, M. ET AL. 'Field performance of elite transgenic maize plants expressing an insecticidal protein derived from Bacillus thuringiensis'
- BIOSCIENCE, BIOTECHNOLOGY, AND BIOCHEMISTRY vol. 56, no. 9 , September 1992 pages 1429 - 1433 YOSHISUE, H. ET AL. 'Effects of Bacillus thuringiensis var. israelensis 20-kDa protein on production of the Bti 130-kDa crystal protein in Escherichia coli'
- PLASMID vol. 16, no. 3 , November 1986 page 230 SHIVAKUMAR, A. ET AL. 'Cloned crystal protein genes express vegetatively in Bacillus subtilis'
- MICROBIOLOGICAL REVIEWS vol. 53, no. 2 , June 1989 , WASHINGTON DC, US pages 242 - 255 HÖFTE, H. & WHITELEY, H. 'Insecticidal crystal proteins of Bacillus thuringiensis'

## Description

The present invention is drawn to methods and compositions for controlling plant and non-plant pests.

Insect pests are a major factor in the loss of the world's commercially important agricultural crops. Broad spectrum chemical pesticides have been used extensively to control or eradicate pests of agricultural importance. There is, however, substantial interest in developing effective alternative pesticides.

Microbial pesticides have played an important role as alternatives to chemical pest control. The most extensively used microbial product is based on the bacterium Bacillus thuringiensis (Bt). Bt is a gram-positive spore forming Bacillus which produces an insecticidal crystal protein (ICP) during sporulation.

Numerous varieties of Bt are known that produce more than 25 different but related ICP's. The ICP's made by Bt are toxic to larvae of certain insects in the orders Lepidoptera, Diptera and Coleoptera. In general, when the ICP is ingested by a susceptible insect the crystal is solubilized and transformed into a toxic moiety by the insect gut proteases. None of the ICP's active against coleopteran larvae have demonstrated significant effects on the genus Diabrotica particularly Diabrotica virgifera virgifera, the western corn rootworm (WCRW) or Diabrotica longicornis barberi, the northern corn rootworm.

Bt is closely related to Bacillus cereus (Bc). A major distinguishing characteristic is the lack of a parasporal crystal in Bc. Bc is a widely distributed bacterium that is commonly found in soil and has been isolated from a variety of foods and drugs. The organism has been implicated in the spoilage of food.

Although Bt has been very useful in controlling insect pests, there is a need to expand the number of potential biological control agents.

The present invention discloses compositions and methods for controlling plant and non-plant pests. Particularly, new pesticidal proteins are disclosed which are isolatable from the vegetative growth stage of Bacillus. Bacillus strains, proteins, and genes encoding the proteins are provided.

The methods and compositions of the invention may be used in a variety of systems for controlling plant and non-plant pests.

One embodiment of the invention is a *Bacillus* strain selected from the group consisting of the strains with accession numbers B-21058, B21060, NRRL B-21225, NRRL B-21226, NRRL B-21227, NRRL B-21228, NRRL B-21229 and NRRL B-21230, wherein said strain produces a pesticidal protein during vegetative growth that is isolatable from liquid culture media, wherein said protein is encoded by a nucleotide sequence that hybridizes to a nucleotide sequence of SEQ ID NO: 1,4,6, 9, 17 or 18, or wherein said protein cross-reacts with antibodies raised against the proteins of SEQ ID NO: 2, 3, 5, 7, 8, or 10-16.

A further embodiment of the invention is a pesticidal protein isolatable during the vegetative growth phase of *Bacillus* spp. or active fragments thereof, wherein said protein is isolatable from liquid culture media, and wherein said protein is encoded by a nucleotide sequence that hybridizes to a nucleotide sequence of SEQ ID NO: 1, 4, 6, 9, 17 or 18, or wherein said protein cross-reacts with antibodies raised against the proteins of SEQ ID NO: 2, 3, 5, 7, 8, or 10 to 16.

A further embodiment of the invention is a nucleotide sequence which encodes a pesticidal protein of the invention.

A further embodiment of the invention is a plant which has been stably transformed with a nucleotide sequence of the invention.

A further embodiment of the invention is a method for producing the pesticidal protein of the invention characterized by the following steps:
a) growing *Bacillus* cells in a culture medium;
b) removing the cells during vegetative growth; and
c) isolating and purifying the pesticidal protein from the liquid culture medium.

Compositions and methods for controlling plant pests are provided. In particular, novel pesticidal proteins are provided which are produced during vegetative growth of Bacillus strains. The proteins are useful as pesticidal agents.

The present invention recognizes that pesticidal proteins are produced during vegetative growth of Bacillus strains. For the purpose of the present invention vegetative growth is defined as that period of time before the onset of sporulation. In the case of Bt, this vegetative growth occurs before production of ICPs. Genes encoding such proteins can be isolated, cloned and transformed into various delivery vehicles for use in pest management programs.

For purposes of the present invention, pests include but are not limited to insects, fungi, bacteria, nematodes, mites, ticks, protozoan pathogens, animal-parasitic liver flukes, and the like. Insect pests include insects selected from the orders Coleoptera, Diptera, Hymenoptera, Lepidoptera, Mallophaga, Homoptera, Hemiptera, Orthroptera, Thysanoptera, Dermaptera, Isoptera, Anoplura, Siphonaptera, Trichoptera, etc.

Tables 1 - 10 gives a list of pests associated with major crop plants and pests of human and veterinary importance. Such pests are included within the scope of the present invention.

**TABLE 1**

| Lepidoptera (Butterflies and Moths) | |
|---|---|
| Maize Ostrinia nubilalis, European corn borer Agrotis ipsilon, black cutworm Helicoverpa zea, corn earworm Spodoptera frugiperda, fall armyworm Diatraea grandiosella, southwestern corn borer Elasmopalpus lignosellus, lesser cornstalk borer Diatraea saccharalis, sugarcane borer | Sunflower Suleima helianthana, sunflower bud moth Homoeosoma electellum, sunflower moth |
| | Cotton Heliothis virescens, cotton boll worm Helicoverpa zea, cotton bollworm Spodoptera exigua, beet armyworm Pectinophora gossypiella, pink bollworm |
| Sorghum Chilo partellus, sorghum borer Spodoptera frugiperda, fall armyworm Helicoverpa zea, corn earworm Elasmopalpus lignosellus, lesser cornstalk borer Feltia subterranea, granulate cutworm | Rice Diatraea saccharalis, sugarcane borer Spodoptera frugiperda, fall armyworm Helicoverpa zea, corn earworm |
| | Soybean Pseudoplusia includens, soybean looper Anticarsia gemmatalis, velvetbean caterpillar Plathypena scabra, green cloverworm Ostrinia nubilalis, European corn borer Agrotis ipsilon, black cutworm Spodoptera exigua, beet armyworm Heliothis virescens, cotton boll worm Helicoverpa zea, cotton bollworm |
| Wheat Pseudaletia unipunctata, army worm Spodoptera frugiperda, fall armyworm Elasmopalpus lignosellus, lesser cornstalk borer Agrotis orthogonia, pale western cutworm Elasmopalpus lignosellus, lesser cornstalk borer | |
| | Barley Ostrinia nubilalis, European corn borer Agrotis ipsilon, black cutworm |

**TABLE 2**

| Coleoptera (Beetles) |
|---|
| Maize |
| Diabrotica virgifera virgifera, western corn rootworm Diabrotica longicornis barberi, northern corn rootworm Diabrotica undecimpunctata howardi, southern com rootworm Melanotus spp., wireworms Cyclocephala borealis, northern masked chafer (white grub) Cyclocephala immaculata, southern masked chafer (white grub) Popillia japonica, Japanese beetle Chaetocnema pulicaria, corn flea beetle Sphenophorus maidis, maize billbug |

| Sorghum |
|---|
| Sorghum Phyllophaga crinita, white grub Eleodes, Conoderus, and Aeolus spp., wireworms Oulema melanopus, cereal leaf beetle Chaetocnema pulicaria, corn flea beetle Sphenophorus maidis, maize billbug |

| Wheat |
|---|
| Oulema melanopus, cereal leaf beetle Hypera punctata, clover leaf weevil Diabrotica undecimpunctata howardi, southern corn rootworm |

| Sunflower |
|---|
| Zygogramma exclamationis, sunflower beetle Bothyrus gibbosus, carrot beetle |

| Cotton |
|---|
| Anthonomus grandis, boll weevil |
| Rice |
| Colaspis brunnea, grape colaspis Lissorhoptrus oryzophilus, rice water weevil Sitophilus oryzae, rice weevil |

| Soybean |
|---|
| Epilachna varivestis, Mexican bean beetle |

**TABLE 3**

| Homoptera (Whiteflies, Aphids etc..) |
|---|
| Maize |
| Rhopalosiphum maidis, corn leaf aphid Anuranhis maidiradicis, corn root aphid |

| Sorghum |
|---|
| Rhopalosiphum maidis, corn leaf aphid Sipha flave, yellow sugarcane aphid |

| Wheat |
|---|
| Russian wheat aphid Schizaphis graminum, greenbug Macrosiphum avenae, English grain aphid |

| Cotton |
|---|
| Aphis gossypii, cotton aphid Pseudatomoscelis seriatus, cotton fleahopper Trialeurodes abutilonea, bandedwinged whitefly |

| Rice |
|---|
| Nephotettix nigropictus, rice leafhopper |

| Soybean |
|---|
| Myzus persicae, green peach aphid Empoasca fabae, potato leafhopper |

| Barley |
|---|
| Schizaphis graminum, greenbug |

| Oil Seed Rape |
|---|
| Brevicoryne brassicae, cabbage aphid |

**TABLE 4**

| Hemiptera (Bugs) |
|---|
| Maize |
| Blissus leucopterus leucopterus, chinch bug |

| Sorghum |
|---|
| Blissus leucopterus leucopterus, chinch bug |

| Cotton |
|---|
| Lygus lineolaris, tarnished plant bug |

| Rice |
|---|
| Blissus leucopterus leucopterus, chinch bug Acrosternum hilare, green stink bug |

| Soybean |
|---|
| Acrostemum hilare, green stink bug |

| Barley |
|---|
| Blissus leucopterus leucopterus, chinch bug Acrostemum hilare, green stink bug Euschistus servus, brown stink bug |

**TABLE 5**

| Orthoptera (Grasshoppers, Crickets, and Cockroaches) |
|---|
| Maize |
| Melanoplus femurrubrum, redlegged grasshopper Melanoplus sanguinipes, migratory grasshopper |

| Wheat |
|---|
| Melanoplus femurrubrum, redlegged grasshopper Melanoplus differentialis, differential grasshopper Melanoplus sanguinipes, migratory grasshopper |

| Cotton |
|---|
| Melanoplus femurrubrum, redlegged grasshopper Melanoplus differentialis, differential grasshopper |

| Soybean |
|---|
| Melanoplus femurrubrum, redlegged grasshopper Melanoplus differentialis, differential grasshopper |

| Structural/Household |
|---|
| Periplaneta americana, American cockroach Blattella germanica, German cockroach Blatta orientalis, oriental cockroach |

**TABLE 6**

| Diptera (Flies and Mosquitoes) |
|---|
| Maize |
| Hylemya platura, seedcorn maggot Agromyza parvicomis, corn blotch leafminer |

| Sorghum |
|---|
| Contarinia sorghicola, sorghum midge |

| Wheat |
|---|
| Mayetiola destructor, Hessian fly Sitodiplosis mosellana, wheat midge Meromyza americana, wheat stem maggot Hylemya coarctata, wheat bulb fly |

| Sunflower |
|---|
| Neolasioptera murtfeldtiana, sunflower seed midge |

| Soybean |
|---|
| Hylemya platura, seedcorn maggot |

| Barley |
|---|
| Hylemya platura, seedcorn maggot Mayetiola destructor, Hessian fly |

| Insects attacking humans and animals and disease carriers |
|---|
| Aedes aegypti, yellowfever mosquito Aedes albopictus, forest day mosquito Phlebotomus papatasii, sand fly Musca domestica, house fly Tabanus atratus, black horse fly Cochliomyia hominivorax, screwworm fly |

**TABLE 7**

| Thysanoptera (Thrips) |
|---|
| Maize |
| Anaphothrips obscurus, grass thrips |

| Wheat |
|---|
| Frankliniella fusca, tobacco thrips |

| Cotton |
|---|
| Thrips tabaci, onion thrips Frankliniella fusca, tobacco thrips |

| Soybean |
|---|
| Sericothrips variabilis, soybean thrips Thrips tabaci, onion thrips |

**TABLE 8**

| Hymenoptera (Sawflies, Ants, Wasps, etc.) |
|---|
| Maize |
| Solenopsis milesta, thief ant |

| Wheat |
|---|
| Cephus cinctus, wheat stem sawfly |

**TABLE 9**

| Other Orders and Representative Species |
|---|
| Dermaptera (Earwigs) |
| Forficula auricularia, European earwig |

| Isoptera (Termites) |
|---|
| Reticulitermes flavipes, eastern subterranean termite |

| Mallophaga (Chewing Lice) |
|---|
| Cuclotogaster heterographa, chicken head louse Bovicola bovis, cattle biting louse |

| Anoplura (Sucking Lice) |
|---|
| Pediculus humanus, head and body louse |

| Siphonaptera (Fleas) |
|---|
| Ctenocephalides felis, cat flea |

**TABLE 10**

| Acari (Mites and Ticks) |
|---|
| Maize |
| Tetranychus urticae, twospotted spider mite |

| Sorghum |
|---|
| Tetranychus cinnabarinus, carmine spider mite Tetranychus urticae, twospotted spider mite |

| Wheat |
|---|
| Aceria tulipae, wheat curl mite |

| Cotton |
|---|
| Tetranychus cinnabarinus, carmine spider mite Tetranychus urticae, twospotted spider mite |

| Soybean |
|---|
| Tetranychus turkestani, strawberry spider mite Tetranychus urticae, twospotted spider mite |

| Barley |
|---|
| Petrobia latens, brown wheat mite |

| Important human and animal Acari |
|---|
| Demacentor variabilis, American dog tick Argas persicus, fowl tick Dermatophagoides farinae, American house dust mite Dermatophagoides pteronyssinus, European house dust mite |

Now that it has been recognized that pesticidal proteins can be isolated from the vegetative growth phase of Bacillus, other strains can be isolated by standard techniques and tested for activity against particular plant and non-plant pests. Generally Bacillus strains can be isolated from any environmental sample, including soil, plant, insect, grain elevator dust, and other sample material, etc., by methods known in the art. See, for example, Travers et al. (1987) Appl. Environ. Microbiol. 53:1263-1266; Saleh et al. (1969) Can J. Microbiol. 15:1101-1104; DeLucca et al. (1981) Can J. Microbiol. 27:865-870; and Norris, et al. (1981) "The genera Bacillus and Sporolactobacillus," In Starr et al. (eds.), The Prokaryotes: A Handbook on Habitats, Isolation, and Identification of Bacteria, Vol. II, Springer-Verlog Berlin Heidelberg. After isolation, strains can be tested for pesticidal activity during vegetative growth. In this manner, new pesticidal proteins and strains can be identified.

Such Bacillus microorganisms which find use in the invention include Bacillus cereus and Bacillus thuringiensis, as well as those Bacillus species listed in Table 11.

**TABLE 11**

| List of Bacillus species | |
|---|---|
| Morphological Group 1 B. megaterium B. cereus* B. cereus var. mycoides B. thuringiensis* B. licheniformis B. subtilis* B. pumilus B. firmus* B. coagulans | **Unassigned Strains** Subgroup A B. apiarus* B. filicolonicus B. thiaminolyticus B. alcalophilus |
| | Subgroup B B. cirroflagellosus B. chitinosporus B. lentus |
| Morphological Group 2 B. polymyxa B. macerans B. circulans B. stearothermophilus B. alvei* B. laterosporus* B. brevis B. pulvifaciens B. popilliae* B. lentimorbus* B. larvae* | Subgroup C B. badius B. aneurinolyticus B. macroides B. freundenreichii |
| | 50 Subgroup D B. pantothenticus B. epiphytus |
| | Subgroup E1 B. aminovorans B. globisporus B. insolitus B. psychrophilus |
| Morphological Group 3 B. sphaericus* B. pasteurii | |
| | Subgroup E2 B. psychrosaccharolyticus B. macquariensis |

| | |
|---|---|
| *=Those Bacillus strains that have been previously found in insects Grouping according to Parry, J.M. et al. (1983) Color Atlas of Bacillus species, Wolfe Medical Publications, London. | |

In accordance with the present invention, the pesticidal proteins produced during vegetative growth can be isolated from Bacillus. In one embodiment, insecticidal proteins produced during vegetative growth, herein after referred to as VIP's (Vegetative Insecticidal Protein), can be isolated. Methods for protein isolation are known in the art. Generally, proteins can be purified by conventional chromatography, including gel-filtration, ion-exchange, and immunoaffinity chromatography, by high-performance liquid chromatography, such as reversed-phase high-performance liquid chromatography, ion-exchange high-performance liquid chromatography, size-exclusion high-performance liquid chromatography, high-performance chromatofocusing and hydrophobic interaction chromatography, etc., by electrophoretic separation, such as one-dimensional gel electrophoresis, two-dimensional gel electrophoresis, etc. Such methods are known in the art. See for example Current Protocols in Molecular Biology, Vols. 1 and 2, Ausubel et al. (eds.), John Wiley & Sons, NY (1988). Additionally, antibodies can be prepared against substantially pure preparations of the protein. See, for example, Radka et al. (1983) J. Immunol. 128:2804; and Radka et al. (1984) Immunogenetics 19:63. Any combination of methods may be utilized to purify protein having pesticidal properties. As the protocol is being formulated, pesticidal activity is determined after each purification step.

Such purification steps will result in a substantially purified protein fraction. By "substantially purified" or "substantially pure" is intended protein which is substantially free of any compound normally associated with the protein in its natural state. "Substantially pure" preparations of protein can be assessed by the absence of other detectable protein bands following SDS-PAGE as determined visually or by densitometry scanning. Alternatively, the absence of other amino-terminal sequences or N-terminal residues in a purified preparation can indicate the level of purity. Purity can be verified by rechromatography of "pure" preparations showing the absence of other peaks by ion exchange, reverse phase or capillary electrophoresis. The terms "substantially pure" or "substantially purified" are not meant to exclude artificial or synthetic mixtures of the proteins with other compounds. The terms are also not meant to exclude the presence of minor impurities which do not interfere with the biological activity of the protein, and which may be present, for example, due to incomplete purification.

Some proteins are single polypeptide chains while many proteins consist of more than one polypeptide chain. Once purified protein is isolated, the protein, or the polypeptides of which it is comprised, can be characterized and sequenced by standard methods known in the art. For example, the purified protein, or the polypeptides of which it is comprised, may be fragmented as with cyanogen bromide, or with proteases such as papain, chymotrypsin, trypsin, lysyl-C endopeptidase, etc. (Oike et al. (1982) J. Biol. Chem. 257:9751-9758; Liu et al. (1983) Int. J. Pept. Protein Res. 21:209-215). The resulting peptides are separated, preferably by HPLC, or by resolution of gels and electroblotting onto PVDF membranes, and subjected to amino acid sequencing. To accomplish this task, the peptides are preferably analyzed by automated sequenators. It is recognized that N-terminal, C-terminal, or internal amino acid sequences can be determined. From the amino acid sequence of the purified protein, a nucleotide sequence can be synthesized which can be used as a probe to aid in the isolation of the gene encoding the pesticidal protein.

It is recognized that the proteins will vary in molecular weight, component peptides, activity against particular pests, and in other characteristics. However, by the methods set forth herein, proteins active against a variety of pests may be isolated and characterized.

Once the purified protein has been isolated and characterized it is recognized that it may be altered in various ways including amino acid substitutions, deletions, and insertions. Methods for such manipulations are generally known in the art. For example, amino acid sequence variants of the pesticidal proteins can be prepared by mutations in the DNA. Such variants will possess the desired pesticidal activity. Obviously, the mutations that will be made in the DNA encoding the variant must not place the sequence out of reading frame and preferably will not create complementary regions that could produce secondary mRNA structure. See, EP Patent Application Publication No. 75,444.

In this manner, the present invention encompasses the pesticidal proteins as well as components and fragments thereof. That is, it is recognized that component polypeptides or fragments of the proteins may be produced which retain pesticidal activity. These fragments include truncated sequences, as well as N-terminal, C-terminal, internal and internally deleted amino acid sequences of the proteins.

Most deletions, insertions, and substitutions of the protein sequence are not expected to produce radical changes in the characteristics of the pesticidal protein. However, when it is difficult to predict the exact effect of the substitution, deletion, or insertion in advance of doing so, one skilled in the art will appreciate that the effect will be evaluated by routine screening assays.

The proteins or other component polypeptides described herein may be used alone or in combination. That is, several proteins may be used to control different insect pests. Additionally, certain of the proteins enhance the activity of the pesticidal proteins. These proteins are referred to herein as "auxiliary proteins." While the mechanism of action is not entirely certain, when the auxiliary protein and the pesticidal protein of interest are together, the insecticidal properties of the pesticidal protein are enhanced several fold.

The pesticidal proteins of the present invention may vary in molecular weight, having component polypeptides at least a molecular weight of 30 kDa or greater, preferably about 50 kDa or greater.

The auxiliary proteins may vary in molecular weight, having at least a molecular weight of about 15 kDa or greater, preferably about 20 kDa or greater. The auxiliary proteins themselves may have component polypeptides.

It is possible that the pesticidal protein and the auxilary protein may be components of a multimeric, pesticidal protein. Such a pesticidal protein which includes the auxiliary proteins as one or more of its component polypeptides may vary in molecular weight, having at least a molecular weight of 50 kDa up to at least 200 kDa, preferably about 100 kDa to 150 kDa.

An auxiliary protein may be used in combination with the pesticidal proteins of the invention to enhance activity. To determine whether the auxiliary protein will affect activity, the pesticidal protein can be expressed alone and in combination with the auxiliary protein and the respective activities compared in feeding assays for increased pesticidal activity.

It may be beneficial to screen strains for potential pesticidal activity by testing activity of the strain alone and in combination with the auxiliary protein. In some instances the auxiliary protein with the native proteins of the strains yields pesticidal activity where none is seen in the absence of the auxiliary protein.

The auxiliary protein can be modified, as described above, by various methods known in the art. Therefore, for purposes of the invention, the term "Vegetative Insecticidal Protein" (VIP) encompasses those proteins produced during vegetative growth which alone or in combination can be used for pesticidal activity. This includes pesticidal proteins, auxiliary proteins and those proteins which demonstrate activity only in the presence of the auxiliary protein or the polypeptide components of these proteins.

It is recognized that there are alternative methods available to obtain the nucleotide and amino acid sequences of the present proteins. For example, to obtain the nucleotide sequence encoding the pesticidal protein, cosmid clones, which express the pesticidal protein, can be isolated from a genomic library. From larger active cosmid clones, smaller subclones can be made and tested for activity. In this manner, clones which express an active pesticidal protein can be sequenced to determine the nucleotide sequence of the gene. Then, an amino acid sequence can be deduced for the protein. For general molecular methods, see, for example, Molecular Cloning, A Laboratory Manual, Second Edition, Vols. 1-3, Sambrook et al. (eds.) Cold Spring Harbor Laboratory Press, Cold Spring Harbor, NY (1989), and the references cited therein.

Once the nucleotide sequences encoding the pesticidal proteins of the invention have been isolated, they can be manipulated and used to express the protein in a variety of hosts including other organisms, including microorganisms and plants.

The pesticidal genes of the invention can be optimized for enhanced expression in plants. See, for example WO 93/07278, EPA 0359472; EPA 0385962; WO 91/16432; Perlak et al (1991) Proc. Natl. Acad, Sci. USA 88:3324-3328; and Murray et al (1989) Nucleic Acids Research 17: 477-498. In this manner, the genes can be synthesized utilizing plant preferred codons. That is the preferred codon for a particular host is the single codon which most frequently encodes that amino acid in that host. The maize preferred codon, for example, for a particular amino acid may be derived from known gene sequences from maize. Maize codon usage for 28 genes from maize plants is found in Murray et al. (1989), Nucleic Acids Research 17:477-498, the disclosure of which is incorporated herein by reference. Synthetic genes could also be made based on the distribution of codons a particular host uses for a particular amino acid.

In this manner, the nucleotide sequences can be optimized for expression in any plant. It is recognized that all or any part of the gene sequence may be optimized or synthetic. That is, synthetic or partially optimized sequences may also be used.

In like manner, the nucleotide sequences can be optimized for expression in any microorganism. For Bacillus preferred codon usage, see, for example US Patent No. 5,024,837 and Johansen et al (1988) Gene 65:293-304.

Methodologies for the construction of plant expression cassettes as well as the introduction of foreign DNA into plants are described in the art. Such expression cassettes may include promoters, terminators, enhancers, leader sequences, introns and other regulatory sequences operably linked to the pesticidal protein coding sequence.

Generally, for the introduction of foreign DNA into plants Ti plasmid vectors have been utilized for the delivery of foreign DNA as well as direct DNA uptake, liposomes, electroporation, micro-injection, and the use of microprojectiles. Such methods had been published in the art. See, for example, Guerche et al., (1987) Plant Science 52:111-116; Neuhause et al., (1987) Theor. Appl. Genet. 75:30-36; Klein et al., (1987) Nature 327: 70-73; Howell et al., (1980) Science 208:1265; Horsch et al., (1985) Science 227: 1229-1231; DeBlock et al., (1989) Plant Physiology 91:694-701; Methods for Plant Molecular Biology (Weissbach and Weissbach, eds.) Academic Press, Inc. (1988); and Methods in Plant Molecular Biology (Schuler and Zielinski, eds.) Academic Press, Inc. (1989). See also US patent Application Serial No. 08/008,374 herein incorporated by reference. See also, EPA 0193259 and EPA 0451878A1. It is understood that the method of transformation will depend upon the plant cell to be transformed.

It is further recognized that the components of the expression cassette may be modified to increase expression. For example, truncated sequences, nucleotide substitutions or other modifications may be employed. See, for example Perlak et al. (1991) Proc. Natl. Acad. Sci. USA 88:3324-3328; Murray et al. (1989) Nucleic Acids Research 17:477-498; and WO 91/16432.

The construct may also include any other necessary regulators such as terminators,(Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639); plant translational consensus sequences (Joshi, C.P., (1987), Nucleic Acids Research, 15:6643-6653), introns (Luehrsen and Walbot, (1991), Mol. Gen. Genet., 225:81-93) and the like, operably linked to the nucleotide sequence. It may be beneficial to include 5' leader sequences in the expression cassette construct. Such leader sequences can act to enhance translation. Translational leaders are known in the art and include:
Picornavirus leaders, for example, EMCV leader (Encephalomyocarditis 5' noncoding region) (Elroy-Stein, O., Fuerst, T.R., and Moss, B. (1989) PNAS USA 86:6126-6130);
Potyvirus leaders, for example, TEV leader (Tobacco Etch Virus) (Allison et al., (1986); MDMV leader (Maize Dwarf Mosaic Virus); Virology, 154:9-20), and
Human immunoglobulin heavy-chain binding protein (BiP), (Macejak, D.G., and Sarnow, P., (1991), Nature, 353:90-94;
Untranslated leader from the coat protein mRNA of alfalfa mosaic virus (AMV RNA 4), (Jobling, S.A., and Gehrke, L., (1987), Nature, 325:622-625;
Tobacco mosaic virus leader (TMV), (Gallie, D.R. et al., (1989), Molecular Biology of RNA, pages 237-256; and
Maize Chlorotic Mottle Virus leader (MCMV) (Lommel, S.A. et al., (1991), Virology, 81:382-385. See also, Della-Cioppa et al., (1987), Plant Physiology, 84:965-968.

A plant terminator may be utilized in the expression cassette. See, Rosenberg et al., (1987), Gene, 56:125; Guerineau et al., (1991), Mol. Gen. Genet., 226:141-144; Proudfoot, (1991), Cell, 64:671-674; Sanfacon et al., (1991), Genes Dev., 5:141-149; Mogen et al., (1990), Plant Cell, 2:1261-1272; Munroe et al., (1990), Gene, 91:151-158; Ballas et al., (1989), Nucleic Acids Res., 17:7891-7903; Joshi et al., (1987), Nucleic Acid Res., 15:9627-9639.

For tissue specific expression, the nucleotide sequences of the invention can be operably linked to tissue specific promoters. See, for example, US Application Serial No. 07/951,715 herein incorporated by reference.

It is recognized that the genes encoding the pesticidal proteins can be used to transform insect pathogenic organisms. Such organisms include Baculoviruses, fungi, protozoa, bacteria and nematodes.

The Bacillus strains of the invention may be used for protecting agricultural crops and products from pests. Alternatively, a gene encoding the pesticide may be introduced via a suitable vector into a microbial host, and said host applied to the environment or plants or animals. Microorganism hosts may be selected which are known to occupy the "phytosphere" (phylloplane, phyllosphere, rhizosphere, and/or rhizoplana) of one or more crops of interest. These microorganisms are selected so as to be capable of successfully competing in the particular environment with the wild-type microorganisms, provide for stable maintenance and expression of the gene expressing the polypeptide pesticide, and, desirably, provide for improved protection of the pesticide from environmental degradation and inactivation.

Such microorganisms include bacteria, algae, and fungi. Of particular interest are microorganisms, such as bacteria, e.g.,Pseudomonas, Erwinia, Serratia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc, and Alcaligenes; fungi, particularly yeast, e.g.,Saccharomyces, Cryptococcus, Kluyveromyces, Sporobolomyces, Rhodotorula, and Aureobasidium. Of particular interest are such phytosphere bacterial species as Pseudomonas syringae, Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacter xyli and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces rosues, S. odorus, Kluyveromyces veronae, and Aureobasidium pollulans. Of particular interest are the pigmented microorganisms.

A number of ways are available for introducing a gene expressing the pesticidal protein into the microorganism host under conditions which allow for stable maintenance and expression of the gene. For example, expression cassettes can be constructed which include the DNA constructs of interest operably linked with the transcriptional and translational regulatory signals for expression of the DNA constructs, and a DNA sequence homologous with a sequence in the host organism, whereby integration will occur, and/or a replication system which is functional in the host, whereby integration or stable maintenance will occur.

Transcriptional and translational regulatory signals include but are not limited to promoter, transcriptional initiation start site, operators, activators, enhancers, other regulatory elements, ribosomal binding sites, an initiation codon, termination signals, and the like. See, for example, US Patent 5,039,523; US Patent No. 4,853,331; EPO 0480762A2; Sambrook et al. supra; Molecular Cloning, a Laboratory Manual, Maniatis et al. (eds) Cold Spring Harbor Laboratory, Cold Spring Harbor, NY (1982); Advanced Bacterial Genetics, Davis et al. (eds.) Cold Spring Harbor Laboratory, Cold Spring Harbor, NY (1980); and the references cited therein.

Suitable host cells, where the pesticide-containing cells will be treated to prolong the activity of the toxin in the cell when the then treated cell is applied to the environment of the target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae and Nitrobacteraceae. Among eukaryotes are fungi, such as Phycomycetes and Ascomycetes, which includes yeast, such a Saccharomyces and Schizosaccharromyces; and Basidiomycetes yeast, such as Rhodotorula, Aureobasidium, Sporobolomyces, and the like.

Characteristics of particular interest in selecting a host cell for purposes of production include ease of introducing the protein gene into the host, availability of expression systems, efficiency of expression, stability of the protein in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., and the like. Specific organisms include Pseudomonas aeurginosa, Pseudomonas fluorescens, Saccharomyces cerevisiae, Bacillus thuringiensis, Escherichia coli, Bacillus subtilis, and the like.

General methods for employing the strains of the invention in pesticide control or in engineering other organisms as pesticidal agents are known in the art. See, for example US Patent No. 5,039,523 and EP 0480762A2.

The Bacillus strains of the invention or the microorganisms which have been genetically altered to contain the pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticides are produced by introducing a heterologous gene into a cellular host. Expression of the heterologous gene results, directly or indirectly, in the intracellular production and maintenance of the pesticide. These cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein.

The active ingredients of the present invention are normally applied in the form of compositions and can be applied to the crop area or plant to be treated, simultaneously or in succession, with other compounds. These compounds can be both fertilizers or micronutrient donors or other preparations that influence plant growth. They can also be selective herbicides, insecticides, fungicides, bactericides, nematicides, mollusicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

Preferred methods of applying an active ingredient of the present invention or an agrochemical composition of the present invention which contains at least one of the pesticidal proteins produced by the bacterial strains of the present invention are leaf application, seed coating and soil application. The number of applications and the rate of application depend on the intensity of infestation by the corresponding pest.

In one embodiment of the invention a Bacillus cereus microorganism has been isolated which is capable of killing Diabrotica virgifera virgifera, and Diabrotica longicornis barberi. The novel B. cereus strain AB78 has been deposited in the Agricultural Research Service, Patent Culture Collection (NRRL), Northern Regional Research Center, 1815 North Univeristy Street, Peoria, IL 61604, USA and given Accession No. NRRL B-21058.

A protein has been substantially purified from the B. cereus strain. Purification of the protein has been verified by SDS-PAGE and biological activity. The protein has a molecular weight of about 60 to about 100 kDa, particularly about 70 to about 90 kDa, more particularly about 80 kDa.

Amino-terminal sequencing has revealed the N-terminal amino-acid sequence to be: NH₂-Lys-Arg-Glu-Ile-Asp-Glu-Asp-Thr-Asp-Thr-Asx-Gly-Asp-Ser-Ile-Pro- (SEQ ID NO:8) where Asx represents either Asp or Asn. The entire amino acid sequence is given in SEQ ID NO:7.

An oligonuleotide probe for the region of the gene encoding amino acids 3-9 of the NH₂-terminus has been generated. The probe was synthesized based on the codon usage of a Bacillus thuringensis (Bt) δ-endotoxin gene. The nucleotide sequence of the oligonucleotide probe used for Southern hybridizations was as follows: where N represents any base.

In addition, the DNA probe for the Bc AB78 VIP-1 gene described herein, permits the screening of any Bacillus strain or other organisms to determine whether the VIP-1 gene (or related gene) is naturally present or whether a particular transformed organism includes the VIP-1 gene.

The invention now being generally described, the same will be better understood by reference to the following detailed examples that are provided for the purpose of illustration and are not to be considered limiting of the invention unless so specified.

### Example 1. AB78 Isolation and Characterization

Bacillus cereus strain AB78 was isolated as a plate contaminant in the laboratory on T3 media (per liter. 3 g tryptone, 2 g tryptose, 1.5 g yeast extract, 0.05 M sodium phosphate (pH 6.8), and 0.005 g MnCl₂; Travers, R.S. 1983). AB78 gave significant activity against western corn rootworm. Antibiotic activity against gram-positive Bacillus spp. was also demonstrated (Table 12).

**Table 12**

| Antibiotic activity of AB78 culture supernatant | | |
|---|---|---|
| Bacteria tested | AB78 | Zone of inhibition(cm) Streptomycin |
| E. coli | 0.0 | 3.0 |
| B. megaterium | 1.1 | 2.2 |
| B. mycoides | 1.3 | 2.1 |
| B. cereus CB | 1.0 | 2.0 |
| B. cereus 11950 | 1.3 | 2.1 |
| B. cereus 14579 | 1.0 | 2.4 |
| B. cereus AB78 | 0.0 | 2.2 |
| Bt var. isrealensis | 1.1 | 2.2 |
| Bt var. tenebrionis | 0.9 | 2.3 |

Morpholgical characteristics of AB78 are as follows:
Vegetative rods straight, 3.1-5.0 mm long and 0.5-2.0 mm wide. Cells with rounded ends, single in short chains. Single subterminal, cylindical-oval, endospore formed per cell. No parasporal crystal formed. Colonies opaque, erose, lobate and flat. No pigments produced. Cells motile. Flagella present.

Growth characteristics of AB78 are as follows:
Facultative anaerobe with optimum growth temperature of21-30°C. Will grow at 15, 20,25,30 and 37°C. Will not grow above 40°C. Grows in 5-7% NaCl.

Table 13 provides the biochemical profile of AB78.

**Table 13**

| Biochemical characteristics of B. cereus strain AB78. | | | |
|---|---|---|---|
| Acid from L-arabinose | - | Methylene blue reoxidized | + |
| Gas from L-arabinose | - | Nitrate reduced | + |
| Acid from D-xylose | - | NO₃ reduced to NO₂ | + |
| Gas from D-xylose | - | VP | + |
| Acid from D-glucose | + | H₂O₂ decomposed | + |
| Gas from D-glucose | - | Indole | - |
| Acid from lactose | - | Tyrosine decomposed | + |
| Gas from lactose | - | Dihydroxiacetone | - |
| Acid from sucrose | - | Litmus milk acid | - |
| Gas from sucrose | - | Litmus milk coagulated | - |
| Acid from D-mannitol | - | Litmus milk alkaline | - |
| Gas from D-mannitol | - | Litmus milk peptonized | - |
| Proprionate utilization | + | + Litmus milk reduced | - |
| Citrate utilization | + | Casein hydrolyzed | + |
| Hippurate hydrolysis | w | Starch hydrolyzed | + |
| Methylene blue reduced | + | Gelatin liquidified | + |
| | | Lecithinase produced | w |
| w= weak reaction | | | |

### Example 2. Bacterial Culture

A subculture of Bc strain AB78 was used to inoculate the following medium, known as TB broth:

| | |
|---|---|
| Tryptone | 12 g/l |
| Yeast Extract | 24 g/l |
| Glycerol | 4 ml/l |
| KH₂PO₄ | 2.1 g/l |
| K₂HPO₄ | 14.7 g/l |
| pH 7.4 | |

The potassium phosphate was added to the autoclaved broth after cooling. Flasks were incubated at 30°C on a rotary shaker at 250 rpm for 24 h.-36 h.

The above procedure can be readily scaled up to large fermentors by procedures well known in the art.

During vegetative growth, usually 24-36 h. after starting the culture, AB78 bacteria were centrifuged from the culture supernatant. The culture supernatant containing the active protein was used in bioassays.

### Example 3. Insect Bioassays

B. cereus strain AB78 was tested against various insects as described below.
**Western, Northern and Southern corn rootworm, Diabrotica virgifera virgifera, D. longcornis barberi and D. undecempunctata howardi, respectively:,** dilutions were made of AB78 culture supernatant grown 24-36 h., mixed with molten artificial diet (Marrone et al. (1985) J. of Economic Entomology 78:290-293) and allowed to solidify. Solidified diet was cut and placed in dishes. Neonate larvae were placed on the diet and held at 30°C. Mortality was recorded after 6 days.

**E. coli clone bioassay: E. coli** was grown overnight in L-Amp100 at 37°C. Ten ml culture was sonicated 3X for 20 sec each. 500 ml of sonicated culture was added to molten western corn rootworm diet.

**Colorado potato beetle Leptinotarsa decemlineata:**-dilutions in Triton X-100 (to give final concentration of 0.1% TX-100) were made of AB78 culture supernatant grown 24-36 h. Five cm² potato leaf pieces were dipped into these dilutions, air dried, and placed on moistened filter paper in plastic dishes. Neonate larvae were placed on the leaf pieces and held at 30°C. Mortality was recorded after 3-5 days.

**Yellow mealworm, Tenebrio molitor:-** dilutions were made of AB78 culture supernatant grown 24-36 h., mixed with molten artificial diet (Bioserv #F9240) and allowed to solidify. Solidified diet was cut and placed in plastic dishes. Neonate larvae were placed on the diet and held at 30°C. Mortality was recorded after 6-8 days.

**European corn borer, black cutworm, tobacco budworm, tobacco hornworm and beet armyworm; Ostrinia nubilalis, Agrotis ipsilon, Heliothis virescens, Manduca sexta and Spodoptera exigua,** respectively: -dilutions, in TX-100 (to give final concentration of 0.1% TX-100), were made of AB78 culture supernatant grown 24-36 hrs. 100 ml was pipetted onto the surface of 18 cm² of solidified artifical diet (Bioserv #F9240) and allowed to air dry. Neonate larvae were then placed onto the surface of the diet and held at 30°C. Mortality was recorded after 3-6 days.

**Northern house mosquito, Culex pipiens:**-dilutions were made of AB78 culture supernatant grown 24-36 h. 100 ml was pipetted into 10 ml water in a 30 ml plastic cup. Third instar larvae were added to the water and held at room temperature. Mortality was recorded after 24-48 hours. The spectrum of entomocidal activity of AB78 is given in Table 14.

**Table 14**

| Activity of AB78 culture supernatant against various insect species | | |
|---|---|---|
| Insect species tested to date | Order | Activity |
| Western corn rootworm (Diabrotica virgifera virgifera) | Col | +++ |
| Northern corn rootworm (Diabrotica longicomis barberi) | Col | +++ |
| Southern corn rootworm (Diabrotica undecimpunctata howardi) | Col | - |
| Colorado potato beetle (Leptinotarsa decemlineata) | Col | - |
| Yellow mealworm (Tenebrio molitor) | Col | - |
| European corn borer (Ostrinia nubilalis) | Lep | - |
| Tobacco budworm (Heliothis virescens) | Lep | - |
| Tobacco hornworm (Manduca sexta) | Lep | - |
| Beet armyworm (Spodoptera exigua) | Lep | - |
| Black cutworm (Agrotis ipsilon) | Lep | - |
| Northern house mosquito (Culex pipiens) | Dip | - |

The newly discovered B. cereus strain AB78 showed a significantly different spectrum of insecticidal activity as compared to known coleopteran active δ-endotoxins from Bt. In particular, AB78 showed more selective activity against beetles than known coleopteran-active Bt strains in that it was specifically active to Diabrotica spp. More specifically, it was most active against D. virgifera virgifera and D. longicornis barberi but not D. undecimpunctata howardi.

A number of Bacillus strains were bioassayed for activity during vegetative growth (Table 15) against western corn rootworm. The results demonstrate that AB78 is unique in that activity against western corn rootworm is not a general phenomenon.

**Table 15**

| Activity of culture supernatants from various Bacillus spp. against western corn rootworm | |
|---|---|
| Bacillus strain | Percent WCRW mortality |
| B. cereus AB78 (Bat.1) | 100 |
| B. cereus AB78 (Bat.2) | 100 |
| B. cereus (Carolina Bio.) | 12 |
| B. cereus ATCC 11950 | 12 |
| B. cereus ATCC 14579 | 8 |
| B. mycoides (Carolina Bio.) | 30 |
| B. popilliae | 28 |
| B. thuringiensis HD135 | 41 |
| B. thuringiensis HD191 | 9 |
| B. thuringiensis GC91 | 4 |
| B. thuringiensis isrealensis | 24 |
| Water Control | 4 |

Specific activity of AB78 against western corn rootworm is provided in Table 16.

**Table 16**

| Activity of AB78 culture supernatant against neonate western corn rootworm | |
|---|---|
| Culture supernatant concentration (µl/ml) | Percent WCRW mortality |
| 100 | 100 |
| 25 | 87 |
| 10 | 80 |
| 5 | 40 |
| 2.5 | 20 |
| 1 | 6 |
| 0 | 0 |

The LC₅₀ was calculated to be 6.2 µl of culture supernatant per ml of western corn rootworm diet.

### Example 4. Isolation and Purification of Corn Rootworm Active Protein from AB78.

Culture media free of cells and debris was made to 70% saturation by the addition of solid ammonium sulfate i.e. (472 g/L). Dissolution was at room temperature followed by cooling in an ice bath and centrifugation at 10,000 x g for thirty minutes to pellet out the precipitated proteins.

The supernatant was discarded and the pellet was dissoved in 1/10 the original volume with 20 mM TRIS-HCI at pH 7.5.

The dissolved pellet was desalted either by dialysis in 20 mM TRIS HCI pH 7.5, or passing through a desalting column.

The desalted material was titrated to pH 3.5 with 20 mM sodium citrate pH 2.5. Following a thirty minute room temperature incubation the solution was centrifuged at 3000 X g for ten minutes. The supernatant at this stage contained the greatest amount of active protein.

Following neutralization of the pH to 7.0 the supernatant was applied to a Mono-Q, anion exchange, column equilibrated with 20 mM TRIS pH 7.5 at a flow rate of 300 mL/min. The column was develped with a stepwise and linear gradient employing 400 mM NaCl in 20 mM TRIS pH 7.5.

Bioassay of the column fractions and SDS-PAGE analysis were used to confirm the active fractions. SDS-PAGE analysis identified the biologically active protein as having a molecular weight in the range of 80 kDa.

### Example 5. Sequence Analysis of the Corn Rootworm Active Protein

The 80 kDa protein isolated by SDS-PAGE was transferred to PVDF membrane and was subjected to amino-terminal sequencing as performed by repetitive Edman cycles on the ABI 470 pulsed-liquid sequencer. Transfer was carried out in 10 mM CAPS buffer with 10% methanol pH 11.0 as follows:
Incubation of the gel following electrophoresis was done in transfer buffer for five minutes.
ProBlott PVDF membrane was wetted with 100% MeOH briefly then equilibrated in transfer buffer.
The sandwich was arranged between foam sponges and filter paper squares with the configuration of Cathode-Gel-Membrane-Anode.
Transfer was performed at 70 V constant voltage for 1 hour.

Following transfer the membrane was rinsed with water and stained for two minutes with 0.25% Coomassie Blue R-250 in 50% MeOH.

Destaining was done with several rinses with 50% MeOH 40% water 10% acetic acid.

Following destaining the membrane was air dried prior to excision of the bands for sequence analysis. A BlottCartridge and appropriate cycles were utilized to achieve maximum efficiency and yield. Data analysis was performed using the model 610 Sequence Analysis software for identifying and quantifying the PTH-amino acid derivatives for each sequential cycle.

The N-terminal sequence was determined to be: where Asx represents Asp or Asn.

### Example 6. Construction of DNA Probe

An oligonucleotide probe for the region of the gene encoding amino acids 3-9 of the N-terminal sequence (Example 5) was generated. The probe was synthesized based on the codon usage of a Bacillus thuringensis (Bt) δ-endotoxin gene. The nucleotide sequence was used as a probe in Southern hybridizations. The oligonucleotide was synthesized using standard procedures and equipment.

### Example 7. Isoelectric Point Determination of the Corn Rootworm Active Protein

Purified protein from step 5 of the purification process was analyzed on a 3-9 pI isoelectric focusing gel using the Phastgel electrophoresis system (Pharmacia). Standard operating procedures for the unit were followed for both the separation and silver staining development procedures. The pI was approximated at about 4.9.

### Example 8. PCR Data On AB78

PCR analysis (See, for example US patent Application Serial No. 08/008,006; and, Carozzi et al. (1991) Appl. Environ. Microbiol. 57(11):3057-3061, herein incorporated by reference.) was used to verify that the B. cereus strain AB78 did not contain any insecticidal crystal protein genes of B. thuringensis or B. sphaericus (Table 17).

**Table 17**

| Bacillus insecticidal crystal protein gene primers tested by PCR against AB78 DNA. | |
|---|---|
| Primers Tested | Product Produced |
| 2 sets specific for CryIIIA | Negative |
| CryIIIB | Negative |
| 2 sets specific for CryIA | Negative |
| CryIA(a) | Negative |
| CryIA(b) specific | Negative |
| CryIB | Negative |
| CryIC specific | Negative |
| CryIE specific | Negative |
| 2 sets specific for B. sphaericus | Negative |
| 2 sets specific for CryIV | Negative |
| Bacillus control (PI-PLC) | Positive |

### Example 9. Cosmid Cloning of Total DNA from B. cereus Strain AB78

The VIP-1 gene was cloned from total DNA prepared from strain AB78 as follows:

### Isolation of AB78 DNA was as follows:

1. Grow bacteria in 10 ml L-broth overnight. (Use 50 ml sterile centrifuge tube)
2. Add 25 ml of fresh L-broth and ampicillin (30 mg/ml).
3. Grow cells 2-6 h. at 30°C with shaking.
4. Spin cells in a 50 ml polypropylene orange cap tube in IEC benchtop clinical centrifuge at 3/4 speed.
5. Resuspend cell pellet in 10 ml TES.
6. Add 30 mg lyzozyme and incubate 2 hrs at 37°C.
7. Add 200 ml 20% SDS and 400 ml Proteinase K (20 mg/ml). Incubate at 37°C.
8. Add 200 ml fresh Proteinase K. Incubate 1 hr. at 55°C. Add 5 ml TES (TES = 50 mM tirs pH 8.0, 100mM EDTA, 15 mM NaCl) to make 15 ml final volume.
9. Phenol extract twice (10 ml phenol, spin at room temperature at 3/4 speed in an IEC benchtop clinical centrifuge). Transfer supernatant (top) to a clean tube with a wide bore pipet.
10. Extract once with 1:1 vol. phenol:chloroform/isoamyl alcohol (24:1 ratio).
11. Precipitate DNA with an equal volume of cold isopropanol; Centrifuge to pellet DNA.
12. Resuspend pellet in 5 ml TE.
13. Precipitate DNA with 0.5 ml 3M NaOAc pH 5.2 and 11 ml 95% ethanol. Place at -20°C for 2 h.
14. "Hook" DNA from tube with a plastic loop, transfer to a microfuge tube, spin, pipet off excess ethanol, dry in vacuo.
15. Resuspend in 0.5 ml TE. Incubate 90 min. at 65°C to help get DNA back into solution.
16. Determine concentration using standard procedures.

### Cosmid Cloning of AB78

All procedures, unless indicated otherwise, were performed according to Stratagene Protocol, Supercos 1 Instruction Manual, Cat. No. 251301.

Generally, the steps were as follows:
A. Sau 3A Partial Digestion of the AB78 DNA.
B. Preparation of Vector DNA
C. Ligation and packaging of DNA
D. Titering the cosmid library
   1. Start a culture of HB101 cells by placing 50 ml of an overnight culture in 5 mls of TB with 0.2% maltose. Incubate 3.5 hrs. at 37°C.
   2. Spin out cells and resuspend in 0.5 mls 10 mM MgSO₄.
   3. Add together:
      100 ml cells
      100 ml diluted packaging mixture
      100 ml 10 mM MgSO₄
      30 ml TB
   4. Adsorb at room temperature for 30 minutes with no shaking.
   5. Add 1 ml TB and mix gently. Incubate 30 minutes at 37°C.
   6. Plate 200 ml onto L-amp plates. Incubate at 37°C overnight.

At least 400 cosmid clones were screened for activity against western corn rootworm as described in Example 3. DNA from 5 active clones and 5 non-active clones were used in Southern hybridizations. Results demonstrated that hybridization using the above described oligonucleotide probe correlated with western corn rootworm activity (Table 18).

Cosmid clones P3-12 and P5-4 have been deposited with the Agricultural Research Service Patent Culture Collecton (NRRL) and given accession nos. B-21061 and B-21059 respectively.

**Table 18**

| **Activity of AB78 cosmid clones against western corn rootworm.** | |
|---|---|
| Clone | Mean percent mortality (N=4) |
| Clones which hybridize with probe | |
| P1-73 | 47 |
| P1-83 | 64 |
| P2-2 | 69 |
| P3-12 | 85 |
| P5-4 | 97 |

| Clones which do not hybridize with probe | |
|---|---|
| P1-2 | 5 |
| P3-8 | 4 |
| P3-9 | 12 |
| P3-18 | 0 |
| P4-6 | 9 |

### Example 10. Identification of a 6 kb region active against western corn rootworm.

DNA from P3-12 was partially digested with restriction enzyme *Sau* 3A, and ligated into the E. coli vector pUC19 and transformed into E. coli. A DNA probe specific for the 80 kDa protein was synthesized by PCR amplification of a portion of P3-12 DNA. The oligonucleotides MK113 and MK117, which hybridize to portions of VIP-1, were synthesized using the partial amino acid sequence of the 80 kDa protein. Plasmid subclones were identified by colony hybridization to the PCR probe, and tested for activity against western corn rootworm. One such clone, PL2, hybridizes to the PCR fragment, and is active against western corn rootworm by the assay previously described.

A 6 kb *Cla* I restriction fragment from PL2 was cloned into the *Sma* I site of the *E.coli*-Bacillus shuttle vector pHT 3101 (Lereclus, D. *et al,* 1989, FEMS Microbiology Letters **60**:211-218) to yield pCIB6201. This construct confers anti-western corn rootworm activity upon both Bacillus and *E.coli* strains, in either orientation. pCIB6022 contains this same 6 kb *Cla* I fragment in pBluescript SK(+) (Stratagene), produces equivalent VIP-1 protein (by western blot), and is also active against western corn rootworm.

The nucleotide sequence of pCIP6022 was determined by the dideoxy termination method of Sanger et al., Proc. Natl. Acad. Sci. USA, 74:5463-5467 (1977), using PRISM Ready Reaction Dye Deoxy Terminator Cycle Sequencing Kits and PRISM Sequenase® Terminator Double-Stranded DNA Sequencing Kit and analyzed on AB1 373 automatic sequencer. The sequence is given in SEQ ID NO:1. pCIB6022 was deposited with the Agricultural Research Service, Patent Culture Collection, (NRRL), Northern Regional Research Center, 1815 North University Street, Peoria, Illinois 61604, USA, and given the Accession No. NRRL B-21222.

### Example 11. Functional dissection of the VIP-1 DNA region.

To confirm that the VIP-1 open reading frame (ORF) is necessary for insecticidal activity a translational frameshift mutation was created in the gene. The restriction enzyme Bgl II recognizes a unique site located 1758 bp into the coding region of VIP-1. pCIB6201 was digested with Bgl II, and the single-stranded ends filled-in with DNA polymerase (Klenow fragmnent) and dNTPS. The plasmid was re-ligated and transformed into E. coli. The resulting plasmid, pCIB6203, contains a four nucleotide insertion in the coding region of VIP-1. pCIB6203 does not confer insecticidal activity, confirming that VIP-1 is an essential component of western corn rootworm activity.

To further define the region necessary to encode VIP-1, subclones of the VIP-1 and VIP-2 (auxiliary protein) region were constructed and tested for their ability to complement the mutation in pCIB6203. pCIB6023 contains the 3.7kb Xba I-EcoRV fragment in pBluescript SK(+) (Stratagene). Western blot analysis indicates that pCIB6023 produces VIP-1 protein of equal size and quantity as clones PL2 and pCIB 6022. pCIB6023 contains the entire gene for the 80kd protein. pCIB6023 was deposited with the Agricultural Research Service, Patent Culture Collection, (NRRL), Northern Regional Research Center, 1815 North University Street, Peoria, Illinois 61604, USA, and given the Accession No. NRRL B-21223.

pCIB6023 shows some western corn rootworm activity. However, the level of activity is less than the activity of pCIB6022. A mixture of cells containing pCIB6203 (VIP-1-mutated, and VIP-2) and cells containing pCIB6023(only VIP-1) shows high activity against western corn rootworm. Thus, pCIB6023 must produce functional VIP-1 gene product, and pCIB6203 must produce a functional VIP-2 gene product. These results suggest a requirement for additional gene product(s) from the VIP-2 region, in combination with VIP-1, to confer maximal western corn rootworm activity. See Table 19.

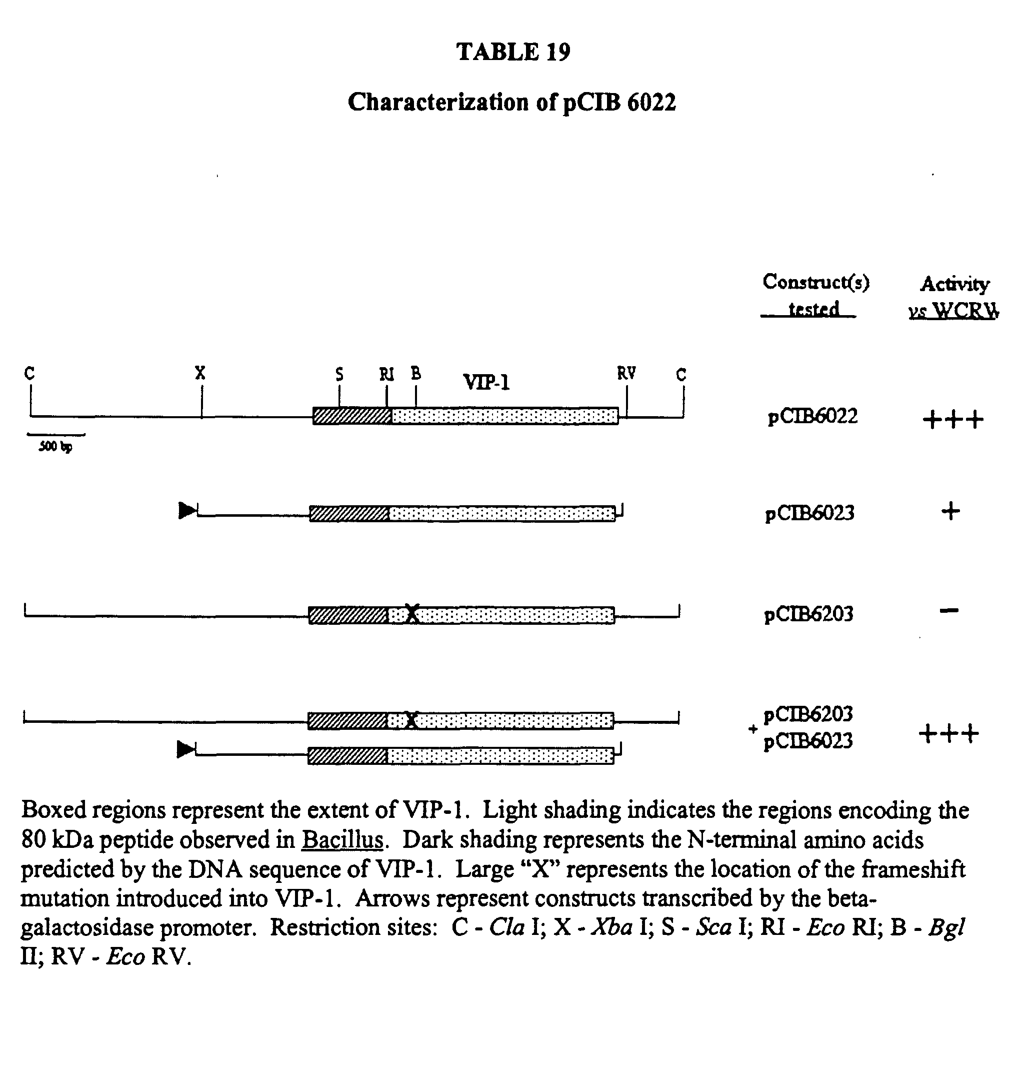

### Example 12. AB78 Antibody Production

Antibody production was initiated in 2 Lewis rats to allow for both the possibility of moving to production of hybridoma cell lines and also to produce enough serum for limited screening of cDNA library. Another factor was the very limited amount of antigen available and the fact that it could only be produced to purity by PAGE and subsequent electrotransfer to nitrocellulose.

Due to the limited availability of antigen on nitrocellulose, the nitrocellulose was emulsified in DMSO and injected into the hind footpads of the animals to elicit B-cell production in the popliteal lymph nodes just upstream. A strong reacting serum was produced by western analysis within the first production bleed. Several subsequent injections and bleeds produced enough serum to accomplish all of the screening required.

Hybridoma production with one of the rats was then initiated. The popliteal lymph node was excised, macerated, and the resulting cells fused with mouise myeloma P3x63Ag8.653. Subsequent cell screening was accomplished as described below. Four initial wells were selected which gave the highest emulsified antigen reaction to be moved to limited dilution cloning. An additional 10 wells were chosen for expansion and cryoperservation.

### Procedure to Emulsify AB78 on nitrocellulose in DMSO for ELISA screening:

After electrotransfer of AB78 samples run on PAGE to nitrocellulose, the reversible strain Ponceaus is used to visualize all protein transferred. The band corresponding to AB78 toxin, previously identified and N-terminal sequenced, is identified and excised from nitrocellulose. Each band is approximately lmmx5mm in size to minimize the amount of nitrocellulose emulsified. A single band is placed in a microfuge tube with 250ul of DMSO and macerated using a plastic pestle (Kontes, Vineland, NJ). To aid in emulsification, the DMSO mixture is heated for 2-3 minutes at 37°C-45°C. Some further maceration might be necessary following heating; however, all of the nitrocellulose should be emulsified. Once the AB78 is emulsified, the sample is placed on ice. In preparation for microtiter plate coating with the emulsified antigen, the sample must be diluted in borate buffered saline as follows: 1:5, 1:10, 1:15, 1:20, 1:30, 1:50, 1:100, and 0. The coating antigen must be prepared fresh immediately prior to use.

ELISA protocol:
1. Coat with AB78/DMSO in BBS. Incubate overnight at 4°C.
2. Wash plate 3X with 1X ELISA wash buffer.
3. Block (1% BSA & 0.05% Tween 20 in PBS) for 30 minutes at Room Temperature.
4. Wash plate 3X with 1X ELISA wash buffer.
5. Add Rat Serum. Incubate 1.5 hours at 37°C.
6. Wash plate 3X with 1X ELISA wash buffer.
7. Add Goat anti-Rat at a conc. of 2ug/ml in ELISA diluent. Incubate 1 hr. at 37°C.
8. Wash plate 3X with 1X ELISA wash buffer.
9. Add Rabbit anti-Goat Alkaline Phosphatase at 2ug/ml in ELISA diluent.
   Incubate 1Hr. at 37°C.
10. Wash 3X with 1X ELISA wash buffer.
11. Add Substrate. Incubate 30 minutes at Room Temperature.
12. Stop with 3N NaOH after 30 minutes.

### Example 13. Activation of insecticidal activity of non-active Bt strains with AB78 VIP clones.

Adding pCIB6203 together with culture supernatant from a Bt strain GC91 produces 100% mortality in Diabrotica virgifera virgifera. Neither pCIB6203 nor GC91 is active on Diabrotica virgifera virgifera by itself. Data are shown below:

| Test material | Percent Diabrotica mortality |
|---|---|
| pCIB6203 | 0 |
| GC91 | 16 |
| pCIB6203 + GC91 | 100 |
| Control | 0 |

### Example 14. Isolation and Biological Activity of B.cereus AB81.

A second B. cereus strain, designated AB81, was isolated from grain bin dust samples by standard methodologies. A subculture of AB81 was grown and prepared for bioassay as described in Example 2. Biological activity was evaluated as described in Example 3. The results are as follows:

| Insect species tested | Percent Mortality |
|---|---|
| Ostrinia nubilalis | 0 |
| Agrotis ipsilon | 0 |
| Diabrotica virgifera virgifera | 55 |

### Example 15. Isolation and Biological Activity of B. thuringiensis AB6.

A B. thuringiensis strain, designated AB6, was isolated from grain bin dust samples by standard methods known in the art. A subculture of AB6 was grown and prepared for bioassay as described in Example 2. Half of the sample was autoclaved 15 minutes to test for the presence of β-exotoxin.

Biological activity was evaluated as described in Example 3. The results are as follows:

| Insect species tested | Percent Mortality |
|---|---|
| Ostrinia nubilalis | 0 |
| Agrotis ipsilon | 100 |
| Agrotis ipsilon (autoclaved sample) | 0 |
| Diabrotica virgifera virgifera | 0 |

Strain AB6 has been deposited in the Agricultural Research Service, Patent Culture Collection (NRRL), Northern Regional Research Center, 1815 North University Street, Peoria, Illinois 61604, USA, and given Accession No. NRRL B-21060.

### Example 16. Isolation and Biological characterization of B. thuringiensis AB88.

A Bt strain, designated AB88, was isolated from grain bin dust samples by standard methodologies. A subculture of AB88 was grown and prepared for bioassay as described in Example 2. Half of the sample was autoclaved 15 minutes to test for the presence of β-exotoxin. Biological activity was evaluated against a number of insect species as described in Example 3. The results are as follows:

| Insect species tested | Order | Percent mortality of culture supernatant | |
|---|---|---|---|
| | | Non-autoclaved | Autoclaved |
| Agrotis ipsilon | Lepidoptera | 100 | 5 |
| Ostrinia nubilalis | Lepidoptera | 100 | 0 |
| Spodoptera frugiperda | Lepidoptera | 100 | 4 |
| Helicoverpa zea | Lepidoptera | 100 | 12 |
| Heliothis virescens | Lepidoptera | 100 | 12 |
| Leptinotarsa decemlineata | Coleoptera | 0 | 0 |
| Diabrotica virgifera virgifera | Coleoptera | 0 | 5 |

Delta-endotoxin crystals were purified from strain AB88 by standard methodologies. No activity from pure crystals was observed when bioassayed against Agrotis ipsilon.

### Example 17. Purification of VIPs from Strain AB88:

Bacterial liquid culture was grown overnight at 30°C in TB media. Cells were spun out and the supernatant kept. Proteins were precipitated with ammonium sulfate (70% saturation), centrifuged and the pellet kept. The pellet was resuspended in the original volume of 20 mM Tris pH 7.5 and dialyzed against the same buffer. AB88 dialysate was more turbid than comparable material from AB78. AB88 proteins have been separated by several different methods following clarification including isoelectric focusing (Rotofor, BioRad, Hercules, CA), precipitation at pH 4.5, ion-exchange chromotography, size exclusion chromatography and ultrafiltration.

European Corn Borer-active protein remained in the pellet obtained by pH 4.5 precipitation of dialysate. When preparative IEF was done on the dialysate using pH 3-10 ampholytes, ECB insecticidal activity was found in all fractions with pH of 7 or greater. SDS-PAGE of these fractions showed protein bands of MW ∼60 kDa and ∼80 kDa. The 60 kDa and 80 kDa bands were separated by anion exchange HPLC on a Poros-Q column (PerSeptive Biosystems, Cambridge, MA). N-terminal sequence was obtained from two fractions containing proteins of slightly differing MW, but both of approximately 60 kDa in size. The sequences obtained were similar to each other and to some δ-endotoxins.
anion exchange fraction 23 (smaller): anion exchange fraction 28 (larger):

When the (active) pH 4.5 pellet was further separated by anion exchange on a Poros-Q column, activity was found only in fractions with a major band of ∼60 kDa.

Black Cutworm-active protein also remained in the pellet when AB88 dialysate was brought down to pH 4.5. In preparative IEF using pH 3-10 ampholytes, actyivity was not found in the ECB-active IEF fractions; instead, it was highest in a fraction of pH 4.5-5.0. Its major components have molecular weights of ∼35 and ∼80 kDa.

The pH 4.5 pellet was separated by anion exchange HPLC to yield fractions containing only the 35 kDa material and fractions containing both 35 kDa and 80 kDa bands.

### Example 18. Characterization of AB88 VIP.

Fractions containing the various lepidopteran active vegetative proteins were generated as described in Example 17. Analysis of active fractions demonstrates that different VIP's are responsible for the different lepidopteran species activity.

The Agrotis ipsilon activity is due to an 80 kDa and or a 35 kDa protein either delivered singly or in combination. These proteins are not related to any δ-endotoxins from Bt as evidenced by the lack of sequence homology of known Bt δ-endotoxin sequences. Also, these proteins are not found in the AB88 δ-endotoxin crystal. N-terminal sequences of the major δ-endotoxin proteins were compared with the N-terminal sequences of the 80 kDa and 35 kDa VIP and reveal no sequence homology. A summary of the results follows:

| Agrotis VIP N-terminal sequences | N-terminal sequence of major δ-endotoxin proteins |
|---|---|
| | 130 kDa MDNNPNINE (SEQ ID NO:14) |
| 80 kDa MNKNNTKLPTRALP (SEQ ID NO:12) | 80 kDa MDNNPNINE (SEQ ID NO:15) |
| | 60 kDa MNVLNSGRTTI (SEQ ID NO:16) |
| 35 kDa ALSENTGKDGGYIVP (SEQ ID NO:13) | |

The Ostrinia nubilalis activity is due to a 60 kDa VIP and the Spodoptera frugiperda activity is due to a VIP of unknown size.

Bacillus thuringiensis strain AB88 has been deposited in the Agricultural Research Service, Patent Culture Collection (NRRL), Northern Regional Research Center, 1815 North University Street, Peoria, Ilinois 61604, USA and given the Accession No. NRRL B-21225.

### Example 19. Isolation and Biological Activity of Other Bacillus sp.

Other Bacillus species have been isolated which produce proteins with insecticidal activity during vegetative growth. These strains were isolated from environmental samples by standard methodologies. Isolates were prepared for bioassay and assayed as described in Examples 2 and 3 respectively. Isolates which produced insecticidal proteins during vegetative growth with activity against Agrotis ipsilon in the bioassay are tabulated below.

| Presence of δ-endotoxin | | |
|---|---|---|
| Bacillus isolate | crystal | Percent mortality |
| AB6 | + | 100 |
| AB53 | - | 80 |
| AB88 | + | 100 |
| AB195 | - | 60 |
| AB211 | - | 70 |
| AB217 | - | 83 |
| AB272 | - | 80 |
| AB279 | - | 70 |
| AB289 | + | 100 |
| AB292 | + | 80 |
| AB294 | - | 100 |
| AB300 | - | 80 |
| AB359 | - | 100 |

Isolates AB289, AB294 and AB359 have been deposited in the Agricultural Research Service, Patent Culture Collection (NRRL), Northern Regional Research Center, 1815 North University Street, Peoria I1 61604, USA and given the Accession Numbers NRRL B-21227, NRRL B-21229, and NRRL B-21226 respectively.

Bacillus isolates which produce insecticidal proteins during vegetative growth with activity against Diabrotica virgifera virgifera are tabulated below.

| Presence of δ-endotoxin | | |
|---|---|---|
| Bacillus isolate | crystal | Percent mortality |
| AB52 | - | 50 |
| AB59 | - | 71 |
| AB68 | + | 60 |
| AB78 | - | 100 |
| AB122 | - | 57 |
| AB218 | - | 64 |
| AB256 | - | 64 |

Isolates AB59 and AB256 have been deposited in the Agricultural Research Service, Patent Culture Collection (NRRL), Northern Regional Research Center, 1815 North University Street, Peoria Illinois 61604, USA, and given the accession numbers NRRL B-21228 and B-21230, respectively.

The following deposits have been made at Agricultural Research Service, Patent Culture Collection (NRRL), Northern Regional Research Center, 1815 North University Street, Peoria, Illinois 61604, U.S.A.:

| | |
|---|---|
| 1. E. coli PL2 | NRRL Accession No. B-21221 |
| 2. E. coli pCIB 6022 | NRRL Accession No. B-21222 |
| 3. E. coli pCIB 6023 | NRRL Accession No. B-21223 |
| 4. Bacillus thuringiensis HD73-78VIP | NRRL Accession No. B-21224 |
| 5. Bacillus thuringiensis AB88 | NRRL Accession No. B-21225 |
| 6. Bacillus thuringiensis AB359 | NRRL Accession No. B-21226 |
| 7. Bacillus thuringiensis AB289 | NRRL Accession No. B-2122z |
| 8. Bacillus sp. AB59 | NRRL Accession No. B-2122u |
| 9. Bacillus sp. AB294 | NRRL Accession No. B-2122i |
| 10. Bacillus sp. AB256 | NRRL Accession No. B-21230 |
| 11. E. coli P5-4 | NRRL Accession No. B-21059 |
| 12. E. coli P3-12 | NRRL Accession No. B-21061 |
| 13. Bacillus cereus AB78 | NRRL Accession No. B-21058 |
| 14. Bacillus thuringiensis AB6 | NRRL Accession No. B-21060 |

### SEQUENCE LISTING

(1) GENERAL INFORMATION:
   (i) APPLICANT:
      (A) NAME: CIBA-GEIGY AG
      (B) STREET: Klybeckstrasse 141
      (C) CITY: Basle
      (E) COUNTRY: Switzerland
      (F) POSTAL CODE (ZIP): CH-4002
      (G) TELEPHONE: (061) 696 11 11
      (H) TELEFAX: (061) 696 79 76

      (A) NAME: Gregory W. Warren
      (B) STREET: 324 Bond Lake Drive
      (C) CITY: Cary
      (D) STATE: NC
      (E) COUNTRY: USA
      (F) POSTAL CODE (ZIP): 27513

      (A) NAME: Michael G. Koziel
      (B) STREET: 509 Carolyn Court
      (C) CITY: Cary
      (D) STATE: NC
      (E) COUNTRY: USA
      (F) POSTAL CODE (ZIP): 27511

      (A) NAME: Martha A. Mullins
      (B) STREET: 104 Countrybrook Lane
      (C) CITY: Youngsville
      (D) STATE: NC
      (E) COUNTRY: USA
      (F) POSTAL CODE (ZIP): 27596

      (A) NAME: Gordon J. Nye
      (B) STREET: 1001 Bray Court
      (C) CITY: Apex
      (D) STATE: NC
      (E) COUNTRY: USA
      (F) POSTAL CODE (ZIP): 27502

      (A) NAME: Brian Carr
      (B) STREET: 110 D Lady's Slipper Ct.
      (C) CITY: Raleigh
      (D) STATE: N.C.
      (E) COUNTRY: U.S.A.
      (F) POSTAL CODE (ZIP): 27606

      (A) NAME: Nalini Manaj Desai
      (B) STREET: 107 Silverwood Lane
      (C) CITY: Cary
      (D) STATE: N.C.
      (E) COUNTRY: U.S.A.
      (F) POSTAL CODE (ZIP): 27511

      (A) NAME: N. Kristy Kostichka
      (B) STREET: 5017 Wineberry Dr.
      (C) CITY: Durham
      (D) STATE: NC
      (E) COUNTRY: USA
      (F) POSTAL CODE (ZIP): 27713
   (ii) TITLE OF INVENTION: Novel Pesticidal Proteins and Strains
   (iii) NUMBER OF SEQUENCES: 18
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: US 08/037,057
      (B) FILING DATE: 25-MAR-1993
(2) INFORMATION FOR SEQ ID NO: 1:
   (i) SEQUENCE CHARACTERISTICS:
      (A) LENGTH: 6106 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: CDS
      (B) LOCATION: 1082..1810
      (D) OTHER INFORMATION: /product= "VIP-2" /label= ORF-1
   (ix) FEATURE:
      (A) NAME/KEY: CDS
      (B) LOCATION: 1925..2470
      (D) OTHER INFORMATION: /product= "VIP-2" /label= ORF-2
   (xi) SEQUENCE DESCRIPTION: SEQ ID NO: 1:
   ATCGATACAA TGTTGTTTTA CTTAGACCGG TAGTCTCTGT AATTTGTTTA ATGCTATATT 60
   CTTTACTTTG ATACATTTTA ATAGCCATTT CAACCTTATC AGTATGTTTT TGTGGTCTTC 120
   CTCCTTTTTT TCCACGAGCT CTAGCTGCGT TTAATCCTGT TTTGGTACGT TCGCTAATAA 180
   TATCTCTTTC TAATTCTGCA ATACTTGCCA TCATTCGAAA GAAGAATTTC CCCATAGCAT 240
   TAGAGGTATC AATGTTGTCA TGAATAGAAA TAAAATCTAC ACCTAGCTCT TTGAATTTTT 300
   CACTTAACTC AATTAGGTGT TTTGTAGAGC GAGAAATTCG ATCAAGTTTG TAAACAACTA 360
   TCTTATCGCC TTTACGTAAT ACTTTTAGCA ACTCTTCGAG TTGAGGGCGC TCTTTTTTTA     420
   TTCCTGTTAT TTTCTCCTGA TATAGCCTTT CTACACCATA TTGTTGCAAA GCATCTATTT     480
   GCATATCGAG ATTTTGTTCT TCTGTGCTGA CACGAGCATA ACCAAAAATC AAATTGGTTT     540
   CACTTCCTAT CTAAATATAT CTATTAAAAT AGCACCAAAA ACCTTATTAA ATTAAAATAA     600
   GGAACTTTGT TTTTGGATAT GGATTTTGGT ACTCAATATG GATGAGTTTT TAACGCTTTT     660
   GTTAAAAAAC AAACAAGTGC CATAAACGGT CGTTTTTGGG ATGACATAAT AAATAATCTG     720
   TTTGATTAAC CTAACCTTGT ATCCTTACAG CCCAGTTTTA TTTGTACTTC AACTGACTGA     780
   ATATGAAAAC AACATGAAGG TTTCATAAAA TTTATATATT TTCCATAACG GATGCTCTAT     840
   CTTTAGGTTA TAGTTAAATT ATAAGAAAAA AACAAACGGA GGGAGTGAAA AAAAGCATCT     900
   TCTCTATAAT TTTACAGGCT CTTTAATAAG AAGGGGGGAG ATTAGATAAT AAATATGAAT     960
   ATCTATCTAT AATTGTTTGC TTCTACAATA ACTTATCTAA CTTTCATATA CAACAACAAA    1020
   ACAGACTAAA TCCAGATTGT ATATTCATTT TCAGTTGTTC CTTTATAAAA TAATTTCATA    1080
   A ATG AAA AGA ATG GAG GGA AAG TTG TTT ATG GTG TCA AAA AAA TTA        1126
     Met Lys Arg Met Glu Gly Lys Leu Phe Met Val Ser Lys Lys Leu
       1               5                  10                  15
   CAA GTA GTT ACT AAA ACT GTA TTG CTT AGT ACA GTT TTC TCT ATA TCT     1174
   Gln Val Val Thr Lys Thr Val Leu Leu Ser Thr Val Phe Ser Ile Ser
                   20                  25                  30
   TTA TTA AAT AAT GAA GTG ATA AAA GCT GAA CAA TTA AAT ATA AAT TCT     1222
   Leu Leu Asn Asn Glu Val Ile Lys Ala Glu Gln Leu Asn Ile Asn Ser
               35                  40                  45
   CAA AGT AAA TAT ACT AAC TTG CAA AAT CTA AAA ATC ACT GAC AAG GTA     1270
   Gln Ser Lys Tyr Thr Asn Leu Gln Asn Leu Lys Ile Thr Asp Lys Val
           50                  55                  60
   GAG GAT TTT AAA GAA GAT AAG GAA AAA GCG AAA GAA TGG GGG AAA GAA     1318
   Glu Asp Phe Lys Glu Asp Lys Glu Lys Ala Lys Glu Trp Gly Lys Glu
       65                  70                  75
   AAA GAA AAA GAG TGG AAA CTA ACT GCT ACT GAA AAA GGA AAA ATG AAT     1366
   Lys Glu Lys Glu Trp Lys Leu Thr Ala Thr Glu Lys Gly Lys Met Asn
   80                  85                  90                  95
   AAT TTT TTA GAT AAT AAA AAT GAT ATA NAG ACA AAT TAT AAA GAA ATT     1414
   Asn Phe Leu Asp Asn Lys Asn Asp Ile Xaa Thr Asn Tyr Lys Glu Ile
                   100                 105                 110
   ACT TTT TCT ATG GCA GGC TCA TTT GAA GAT GAA ATA AAA GAT TTA AAA     1462
   Thr Phe Ser Met Ala Gly Ser Phe Glu Asp Glu Ile Lys Asp Leu Lys
               115                 120                 125
   GAA ATT GAT AAG ATG TTT GAT AAA ACC AAT CTA TCA AAT TCT ATT ATC     1510
   Glu Ile Asp Lys Met Phe Asp Lys Thr Asn Leu Ser Asn Ser Ile Ile
           130                 135                 140
   ACC TAT AAA AAT GTG GAA CCG ACA ACA ATT GGA TTT AAT AAA TCT TTA        1558
   Thr Tyr Lys Asn Val Glu Pro Thr Thr Ile Gly Phe Asn Lys Ser Leu
       145                 150                 155
   ACA GAA GGT AAT ACG ATT AAT TCT GAT GCA ATG GCA CAG TTT AAA GAA        1606
   Thr Glu Gly Asn Thr Ile Asn Ser Asp Ala Met Ala Gln Phe Lys Glu
   160                 165                 170                 175
   CAA TTT TTA GAT AGG GAT ATT AAG TTT GAT AGT TAT CTA GAT ACG CAT        1654
   Gln Phe Leu Asp Arg Asp Ile Lys Phe Asp Ser Tyr Leu Asp Thr His
                   180                 185                 190
   TTA ACT GCT CAA CAA GTT TCC AGT AAA GAA AGA GTT ATT TTG AAG GTT        1702
   Leu Thr Ala Gln Gln Val Ser Ser Lys Glu Arg Val Ile Leu Lys Val
               195                 200                 205
   ACG GTT CCG AGT GGG AAA GGT TCT ACT ACT CCA ACA AAA GCA GGT GTC        1750
   Thr Val Pro Ser Gly Lys Gly Ser Thr Thr Pro Thr Lys Ala Gly Val
           210                 215                 220
   ATT TTA AAT AAT AGT GAA TAC AAA ATG CTC ATT GAT AAT GGG TAT ATG        1798
   Ile Leu Asn Asn Ser Glu Tyr Lys Met Leu Ile Asp Asn Gly Tyr Met
       225                 230                 235
   GTC CAT GTA GAT TAAGGTATCA AAAGTGGTGA AAAAAGGGGG TGGAGTGCCT           1850
   Val His Val Asp
   240
   TACAAATTGA AGGGACTTTA AAAAAGAGTC TTGACTTTAA AAATGATATA AATGCTGAAG      1910
   CGCATAGCTG GGGT ATG AAG AAT TAT GAA GAG TGG GCT AAA GAT TTA ACC        1960
                   Met Lys Asn Tyr Glu Glu Trp Ala Lys Asp Leu Thr
                     1               5                  10
   GAT TCG CAA AGG GAA GCT TTA GAT GGG TAT GCT AGG CAA GAT TAT AAA        2008
   Asp Ser Gln Arg Glu Ala Leu Asp Gly Tyr Ala Arg Gln Asp Tyr Lys
           15                  20                  25
   GAA ATC AAT AAT TAT TTA AGA AAT CAA GGC GGA AGT GGA AAT GAA AAA        2056
   Glu Ile Asn Asn Tyr Leu Arg Asn Gln Gly Gly Ser Gly Asn Glu Lys
       30                  35                  40
   CTA GAT GCT CAA ATA AAA AAT ATT TCT GAT GCT TTA GGG AAG AAA CCA        2104
   Leu Asp Ala Gln Ile Lys Asn Ile Ser Asp Ala Leu Gly Lys Lys Pro
   45                  50                  55                  60
   ATA CCG GAA AAT ATT ACT GTG TAT AGA TGG TGT GGC ATG CCG GAA TTT        2152
   Ile Pro Glu Asn Ile Thr Val Tyr Arg Trp Cys Gly Met Pro Glu Phe
                   65                  70                  75
   GGT TAT CAA ATT AGT GAT CCG TTA CCT TCT TTA AAA GAT TTT GAA GAA        2200
   Gly Tyr Gln Ile Ser Asp Pro Leu Pro Ser Leu Lys Asp Phe Glu Glu
               80                  85                  90
   CAA TTT TTA AAT ACA ATC AAA GAA GAC AAA GGA TAT ATG AGT ACA AGC        2248
   Gln Phe Leu Asn Thr Ile Lys Glu Asp Lys Gly Tyr Met Ser Thr Ser
           95                  100                 105
   TTA TCG AGT GAA CGT CTT GCA GCT TTT GGA TCT AGA AAA ATT ATA TTA        2296
   Leu Ser Ser Glu Arg Leu Ala Ala Phe Gly Ser Arg Lys Ile Ile Leu
       110                 115                 120
   CGA TTA CAA GTT CCG AAA GGA AGT ACG GGT GCG TAT TTA AGT GCC ATT      2344
   Arg Leu Gln Val Pro Lys Gly Ser Thr Gly Ala Tyr Leu Ser Ala Ile
   125                 130                 135                 140
   GGT GGA TTT GCA AGT GAA AAA GAG ATC CTA CTT GAT AAA GAT AGT AAA      2392
   Gly Gly Phe Ala Ser Glu Lys Glu Ile Leu Leu Asp Lys Asp Ser Lys
                   145                 150                 155
   TAT CAT ATT GAT AAA GTA ACA GAG GTA ATT ATT AAA GGT GTT AAG CGA      2440
   Tyr His Ile Asp Lys Val Thr Glu Val Ile Ile Lys Gly Val Lys Arg
               160                 165                 170
   TAT GTA GTG GAT GCA ACA TTA TTA ACA AAT TAAGGAGATG AAAAATATGA        2490
   Tyr Val Val Asp Ala Thr Leu Leu Thr Asn
           175                 180
   AGAAAAAGTT AGCAAGTGTT GTAACGTGTA CGTTATTAGC TCCTATGTTT TTGAATGGAA    2550
   ATGTGAATGC TGTTTACGCA GACAGCAAAA CAAATCAAAT TTCTACAACA CAGAAAAATC    2610
   AACAGAAAGA GATGGACCGA AAAGGATTAC TTGGGTATTA TTTCAAAGGA AAAGATTTTA    2670
   GTAATCTTAC TATGTTTGCA CCGACACGTG ATAGTACTCT TATTTATGAT CAACAAACAG    2730
   CAAATAAACT ATTAGATAAA AAACAACAAG AATATCAGTC TATTCGTTGG ATTGGTTTGA    2790
   TTCAGAGTAA AGAAACGGGA GATTTCACAT TTAACTTATC TGAGGATGAA CAGGCAATTA    2850
   TAGAAATCAA TGGGAAAATT ATTTCTAATA AAGGGAAAGA AAAGCAAGTT GTCCATTTAG    2910
   AAAAAGGAAA ATTAGTTCCA ATCAAAATAG AGTATCAATC AGATACAAAA TTTAATATTG    2970
   ACAGTAAAAC ATTTAAAGAA CTTAAATTAT TTAAAATAGA TAGTCAAAAC CAACCCCAGC    3030
   AAGTCCAGCA AGATGAACTG AGAAATCCTG AATTTAACAA GAAAGAATCA CAGGAATTCT    3090
   TAGCGAAACC ATCGAAAATA AATCTTTTCA CTCAAMAAAT GAAAAGGGAA ATTGATGAAG    3150
   ACACGGATAC GGATGGGGAC TCTATTCCTG ACCTTTGGGA AGAAAATGGG TATACGATTC    3210
   AMAATAGAAT CGCTGTAAAG TGGGACGATT CTCTAGCAAG TAAAGGGTAT ACGAAATTTG    3270
   TTTCAAATCC ACTAGAAAGT CACACAGTTG GTGATCCTTA TACAGATTAT GAAAAGGCAG    3330
   CAAGAGATCT AGATTTGTCA AATGCAAAGG AAACGTTTAA CCCATTGGTA GCTGCTTTTC    3390
   CAAGTGTGAA TGTTAGTATG GAAAAGGTGA TATTATCACC AAATGAAAAT TTATCCAATA    3450
   GTGTAGAGTC TCATTCATCC ACGAATTGGT CTTATACAAA TACAGAAGGT GCTTCTGTTG    3510
   AAGCGGGGAT TGGACCAAAA GGTATTTCGT TCGGAGTTAG CGTAAACTAT CAACACTCTG    3570
   AAACAGTTGC ACAAGAATGG GGAACATCTA CAGGAAATAC TTCGCAATTC AATACGGCTT    3630
   CAGCGGGATA TTTAAATGCA AATGTTCGAT ATAACAATGT AGGAACTGGT GCCATCTACG    3690
   ATGTAAAACC TACAACAAGT TTTGTATTAA ATAACGATAC TATCGCAACT ATTACGGCGA    3750
   AATCTAATTC TACAGCCTTA AATATATCTC CTGGAGAAAG TTACCCGAAA AAAGGACAAA 3810
   ATGGAATCGC AATAACATCA ATGGATGATT TTAATTCCCA TCCGATTACA TTAAATAAAA 3870
   AACAAGTAGA TAATCTGCTA AATAATAAAC CTATGATGTT GGAAACAAAC CAAACAGATG 3930
   GTGTTTATAA GATAAAAGAT ACACATGGAA ATATAGTAAC TGGCGGAGAA TGGAATGGTG 3990
   TCATACAACA AATCAAGGCT AAAACAGCGT CTATTATTGT GGATGATGGG GAACGTGTAG 4050
   CAGAAAAACG TGTAGCGGCA AAAGATTATG AAAATCCAGA AGATAAAACA CCGTCTTTAA 4110
   CTTTAAAAGA TGCCCTGAAG CTTTCATATC CAGATGAAAT AAAAGAAATA GAGGGATTAT 4170
   TATATTATAA AAACAAACCG ATATACGAAT CGAGCGTTAT GACTTACTTA GATGAAAATA 4230
   CAGCAAAAGA AGTGACCAAA CAATTAAATG ATACCACTGG GAAATTTAAA GATGTAAGTC 4290
   ATTTATATGA TGTAAAACTG ACTCCAAAAA TGAATGTTAC AATCAAATTG TCTATACTTT 4350
   ATGATAATGC TGAGTCTAAT GATAACTCAA TTGGTAAATG GACAAACACA AATATTGTTT 4410
   CAGGTGGAAA TAACGGAAAA AAACAATATT CTTCTAATAA TCCGGATGCT AATTTGACAT 4470
   TAAATACAGA TGCTCAAGAA AAATTAAATA AAAATCGTGA CTATTATATA AGTTTATATA 4530
   TGAAGTCAGA AAAAAACACA CAATGTGAGA TTACTATAGA TGGGGAGATT TATCCGATCA 4590
   CTACAAAAAC AGTGAATGTG AATAAAGACA ATTACAAAAG ATTAGATATT ATAGCTCATA 4650
   ATATAAAAAG TAATCCAATT TCTTCACTTC ATATTAAAAC GAATGATGAA ATAACTTTAT 4710
   TTTGGGATGA TATTTCTATA ACAGATGTAG CATCAATAAA ACCGGAAAAT TTAACAGATT 4770
   CAGAAATTAA ACAGATTTAT AGTAGGTATG GTATTAAGTT AGAAGATGGA ATCCTTATTG 4830
   ATAAAAAAGG TGGGATTCAT TATGGTGAAT TTATTAATGA AGCTAGTTTT AATATTGAAC 4890
   CATTGCCAAA TTATGTGACC AAATATGAAG TTACTTATAG TAGTGAGTTA GGACCAAACG 4950
   TGAGTGACAC ACTTGAAAGT GATAAAATTT ACAAGGATGG GACAATTAAA TTTGATTTTA 5010
   CCAAATATAG TAAAAATGAA CAAGGATTAT TTTATGACAG TGGATTAAAT TGGGACTTTA 5070
   AAATTAATGC TATTACTTAT GATGGTAAAG AGATGAATGT TTTTCATAGA TATAATAAAT 5130
   AGTTATTATA TCTATGAAGC TGGTGCTAAA GATAGTGTAA AAGTTAATAT ACTGTAGGAT 5190
   TGTAATAAAA GTAATGGAAT TGATATCGTA CTTTGGAGTG GGGGATACTT TGTAAATAGT 5250
   TCTATCAGAA ACATTAGACT AAGAAAAGTT ACTACCCCCA CTTGAAAATG AAGATTCAAC 5310
   TGATTACAAA CAACCTGTTA AATATTATAA GGTTTTAACA AAATATTAAA CTCTTTATGT 5370
   TAATACTGTA ATATAAAGAG TTTAATTGTA TTCAAATGAA GCTTTCCCAC AAAATTAGAC 5430
   TGATTATCTA ATGAAATAAT CAGTCTAATT TTGTAGAACA GGTCTGGTAT TATTGTACGT 5490
   GGTCACTAAA AGATATCTAA TATTATTGGG CAAGGCGTTC CATGATTGAA TCCTCGAATG 5550
   TCTTGCCCTT TTCATTTATT TAAGAAGGAT TGTGGAGAAA TTATGGTTTA GATAATGAAG    5610
   AAAGACTTCA CTTCTAATTT TTGATGTTAA ATAAATCAAA ATTTGGCGAT TCACATTGTT    5670
   TAATCCACTG ATAAAACATA CTGGAGTGTT CTTAAAAAAT CAGCTTTTTT CTTTATAAAA    5730
   TTTTGCTTAG CGTACGAAAT TCGTGTTTTG TTGGTGGGAC CCCATGCCCA TCAACTTAAG    5790
   AGTAAATTAG TAATGAACTT TCGTTCATCT GGATTAAAAT AACCTCAAAT TAGGACATGT    5850
   TTTTAAAAAT AAGCAGACCA AATAAGCCTA GAATAGGTAT CATTTTTAAA AATTATGCTG    5910
   CTTTCTTTTG TTTTCCAAAT CCATTATACT CATAAGCAAC ACCCATAATG TCAAAGACTG    5970
   TTTTTGTCTC ATATCGATAA GCTTGATATC GAATTCCTGC AGCCCGGGGG ATCCACTAGT    6030
   TCTAGAGCGG CCGCCACCGC GGTGGAGCTC CAGCTTTTGT TCCCTTTAGT GAGGGTTAAG    6090
   TTCGAGCTTG TCGTGG                                                    6106
(2) INFORMATION FOR SEQ ID NO: 2:
   (i) SEQUENCE CHARACTERISTICS:
      (A) LENGTH: 243 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Lys Arg Met Glu Gly Lys Leu Phe Met Val Ser Lys Lys Leu Gln
    1               5                  10                  15
   Val Val Thr Lys Thr Val Leu Leu Ser Thr Val Phe Ser Ile Ser Leu
               20                  25                  30
   Leu Asn Asn Glu Val Ile Lys Ala Glu Gln Leu Asn Ile Asn Ser Gln
           35                  40                  45
   Ser Lys Tyr Thr Asn Leu Gln Asn Leu Lys Ile Thr Asp Lys Val Glu
       50                  55                  60
   Asp Phe Lys Glu Asp Lys Glu Lys Ala Lys Glu Trp Gly Lys Glu Lys
   65                  70                  75                      80
   Glu Lys Glu Trp Lys Leu Thr Ala Thr Glu Lys Gly Lys Met Asn Asn
                   85                  90                  95
   Phe Leu Asp Asn Lys Asn Asp Ile Xaa Thr Asn Tyr Lys Glu Ile Thr
               100                 105                 110
   Phe Ser Met Ala Gly Ser Phe Glu Asp Glu Ile Lys Asp Leu Lys Glu
           115                 120                 125
   Ile Asp Lys Met Phe Asp Lys Thr Asn Leu Ser Asn Ser Ile Ile Thr
       130                 135                 140
   Tyr Lys Asn Val Glu Pro Thr Thr Ile Gly Phe Asn Lys Ser Leu Thr
   145                 150                 155                 160
   Glu Gly Asn Thr Ile Asn Ser Asp Ala Met Ala Gln Phe Lys Glu Gln
                   165                 170                 175
   Phe Leu Asp Arg Asp Ile Lys Phe Asp Ser Tyr Leu Asp Thr His Leu
               180                 185                 190
   Thr Ala Gln Gln Val Ser Ser Lys Glu Arg Val Ile Leu Lys Val Thr
           195                 200                 205
   Val Pro Ser Gly Lys Gly Ser Thr Thr Pro Thr Lys Ala Gly Val Ile
       210                 215                 220
   Leu Asn Asn Ser Glu Tyr Lys Met Leu Ile Asp Asn Gly Tyr Met Val
   225                 230                 235                 240
   His Val Asp
(2) INFORMATION FOR SEQ ID NO: 3:
   (i) SEQUENCE CHARACTERISTICS:
      (A) LENGTH: 182 amino acids
      (B) TYPE: amino acid
      (D) TOPOLOGY: linear
   (ii) MOLECULE TYPE: protein
   (xi) SEQUENCE DESCRIPTION: SEQ ID NO: 3:
   Met Lys Asn Tyr Glu Glu Trp Ala Lys Asp Leu Thr Asp Ser Gln Arg
    1               5                  10                  15
   Glu Ala Leu Asp Gly Tyr Ala Arg Gln Asp Tyr Lys Glu Ile Asn Asn
               20                  25                  30
   Tyr Leu Arg Asn Gln Gly Gly Ser Gly Asn Glu Lys Leu Asp Ala Gln
           35                  40                  45
   Ile Lys Asn Ile Ser Asp Ala Leu Gly Lys Lys Pro Ile Pro Glu Asn
       50                  55                  60
   Ile Thr Val Tyr Arg Trp Cys Gly Met Pro Glu Phe Gly Tyr Gln Ile
   65                  70                  75                  80
   Ser Asp Pro Leu Pro Ser Leu Lys Asp Phe Glu Glu Gln Phe Leu Asn
                   85                  90                  95
   Thr Ile Lys Glu Asp Lys Gly Tyr Met Ser Thr Ser Leu Ser Ser Glu
               100                 105                 110
   Arg Leu Ala Ala Phe Gly Ser Arg Lys Ile Ile Leu Arg Leu Gln Val
           115                 120                 125
   Pro Lys Gly Ser Thr Gly Ala Tyr Leu Ser Ala Ile Gly Gly Phe Ala
       130                 135                 140
   Ser Glu Lys Glu Ile Leu Leu Asp Lys Asp Ser Lys Tyr His Ile Asp
   145                 150                 155                 160
   Lys Val Thr Glu Val Ile Ile Lys Gly Val Lys Arg Tyr Val Val Asp
                   165                 170                 175
   Ala Thr Leu Leu Thr Asn
               180 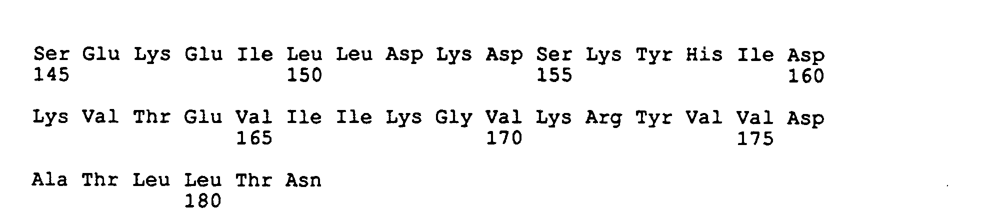
(2) INFORMATION FOR SEQ ID NO: 4:
   (i) SEQUENCE CHARACTERISTICS:
      (A) LENGTH: 2655 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: CDS
      (B) LOCATION: 1..2652
      (C) IDENTIFICATION METHOD: experimental
      (D) OTHER INFORMATION: /product= "100 kDa protein VIP-1" /evidence= EXPERIMENTAL
   (xi) SEQUENCE DESCRIPTION: SEQ ID NO: 4:
   ATG AAA AAT ATG AAG AAA AAG TTA GCA AGT GTT GTA ACG TGT ACG TTA        48
   Met Lys Asn Met Lys Lys Lys Leu Ala Ser Val Val Thr Cys Thr Leu
    1               5                  10                  15
   TTA GCT CCT ATG TTT TTG AAT GGA AAT GTG AAT GCT GTT TAC GCA GAC        96
   Leu Ala Pro Met Phe Leu Asn Gly Asn Val Asn Ala Val Tyr Ala Asp
               20                  25                  30
   AGC AAA ACA AAT CAA ATT TCT ACA ACA CAG AAA AAT CAA CAG AAA GAG       144
   Ser Lys Thr Asn Gln Ile Ser Thr Thr Gln Lys Asn Gln Gln Lys Glu
           35                  40                  45
   ATG GAC CGA AAA GGA TTA CTT GGG TAT TAT TTC AAA GGA AAA GAT TTT       192
   Met Asp Arg Lys Gly Leu Leu Gly Tyr Tyr Phe Lys Gly Lys Asp Phe
       50                  55                  60
   AGT AAT CTT ACT ATG TTT GCA CCG ACA CGT GAT AGT ACT CTT ATT TAT       240
   Ser Asn Leu Thr Met Phe Ala Pro Thr Arg Asp Ser Thr Leu Ile Tyr
   65                  70                  75                  80
   GAT CAA CAA ACA GCA AAT AAA CTA TTA GAT AAA AAA CAA CAA GAA TAT       288
   Asp Gln Gln Thr Ala Asn Lys Leu Leu Asp Lys Lys Gln Gln Glu Tyr
                       85                  90                  95
   CAG TCT ATT CGT TGG ATT GGT TTG ATT CAG AGT AAA GAA ACG GGA GAT      336
   Gln Ser Ile Arg Trp Ile Gly Leu Ile Gln Ser Lys Glu Thr Gly Asp
               100                 105                 110
   TTC ACA TTT AAC TTA TCT GAG GAT GAA CAG GCA ATT ATA GAA ATC AAT      384
   Phe Thr Phe Asn Leu Ser Glu Asp Glu Gln Ala Ile Ile Glu Ile Asn
           115                 120                 125
   GGG AAA ATT ATT TCT AAT AAA GGG AAA GAA AAG CAA GTT GTC CAT TTA      432
   Gly Lys Ile Ile Ser Asn Lys Gly Lys Glu Lys Gln Val Val His Leu
       130                 135                 140
   GAA AAA GGA AAA TTA GTT CCA ATC AAA ATA GAG TAT CAA TCA GAT ACA      480
   Glu Lys Gly Lys Leu Val Pro Ile Lys Ile Glu Tyr Gln Ser Asp Thr
   145                 150                 155                 160
   AAA TTT AAT ATT GAC AGT AAA ACA TTT AAA GAA CTT AAA TTA TTT AAA      528
   Lys Phe Asn Ile Asp Ser Lys Thr Phe Lys Glu Leu Lys Leu Phe Lys
                   165                 170                 175
   ATA GAT AGT CAA AAC CAA CCC CAG CAA GTC CAG CAA GAT GAA CTG AGA      576
   Ile Asp Ser Gln Asn Gln Pro Gln Gln Val Gln Gln Asp Glu Leu Arg
               180                 185                 190
   AAT CCT GAA TTT AAC AAG AAA GAA TCA CAG GAA TTC TTA GCG AAA CCA      624
   Asn Pro Glu Phe Asn Lys Lys Glu Ser Gln Glu Phe Leu Ala Lys Pro
           195                 200                 205
   TCG AAA ATA AAT CTT TTC ACT CAA MAA ATG AAA AGG GAA ATT GAT GAA      672
   Ser Lys Ile Asn Leu Phe Thr Gln Xaa Met Lys Arg Glu Ile Asp Glu
       210                 215                 220
   GAC ACG GAT ACG GAT GGG GAC TCT ATT CCT GAC CTT TGG GAA GAA AAT      720
   Asp Thr Asp Thr Asp Gly Asp Ser Ile Pro Asp Leu Trp Glu Glu Asn
   225                 230                 235                 240
   GGG TAT ACG ATT CAM AAT AGA ATC GCT GTA AAG TGG GAC GAT TCT CTA      768
   Gly Tyr Thr Ile Xaa Asn Arg Ile Ala Val Lys Trp Asp Asp Ser Leu
                   245                 250                 255
   GCA AGT AAA GGG TAT ACG AAA TTT GTT TCA AAT CCA CTA GAA AGT CAC      816
   Ala Ser Lys Gly Tyr Thr Lys Phe Val Ser Asn Pro Leu Glu Ser His
               260                 265                 270
   ACA GTT GGT GAT CCT TAT ACA GAT TAT GAA AAG GCA GCA AGA GAT CTA      864
   Thr Val Gly Asp Pro Tyr Thr Asp Tyr Glu Lys Ala Ala Arg Asp Leu
           275                 280                 285
   GAT TTG TCA AAT GCA AAG GAA ACG TTT AAC CCA TTG GTA GCT GCT TTT      912
   Asp Leu Ser Asn Ala Lys Glu Thr Phe Asn Pro Leu Val Ala Ala Phe
       290                 295                 300
   CCA AGT GTG AAT GTT AGT ATG GAA AAG GTG ATA TTA TCA CCA AAT GAA      960
   Pro Ser Val Asn Val Ser Met Glu Lys Val Ile Leu Ser Pro Asn Glu
   305                 310                 315                 320
   AAT TTA TCC AAT AGT GTA GAG TCT CAT TCA TCC ACG AAT TGG TCT TAT     1008
   Asn Leu Ser Asn Ser Val Glu Ser His Ser Ser Thr Asn Trp Ser Tyr
                   325                 330                 335
   ACA AAT ACA GAA GGT GCT TCT GTT GAA GCG GGG ATT GGA CCA AAA GGT      1056
   Thr Asn Thr Glu Gly Ala Ser Val Glu Ala Gly Ile Gly Pro Lys Gly
               340                 345                 350
   ATT TCG TTC GGA GTT AGC GTA AAC TAT CAA CAC TCT GAA ACA GTT GCA      1104
   Ile Ser Phe Gly Val Ser Val Asn Tyr Gln His Ser Glu Thr Val Ala
           355                 360                 365
   CAA GAA TGG GGA ACA TCT ACA GGA AAT ACT TCG CAA TTC AAT ACG GCT      1152
   Gln Glu Trp Gly Thr Ser Thr Gly Asn Thr Ser Gln Phe Asn Thr Ala
       370                 375                 380
   TCA GCG GGA TAT TTA AAT GCA AAT GTT CGA TAT AAC AAT GTA GGA ACT      1200
   Ser Ala Gly Tyr Leu Asn Ala Asn Val Arg Tyr Asn Asn Val Gly Thr
   385                 390                 395                 400
   GGT GCC ATC TAC GAT GTA AAA CCT ACA ACA AGT TTT GTA TTA AAT AAC      1248
   Gly Ala Ile Tyr Asp Val Lys Pro Thr Thr Ser Phe Val Leu Asn Asn
                   405                 410                 415
   GAT ACT ATC GCA ACT ATT ACG GCG AAA TCT AAT TCT ACA GCC TTA AAT      1296
   Asp Thr Ile Ala Thr Ile Thr Ala Lys Ser Asn Ser Thr Ala Leu Asn
               420                 425                 430
   ATA TCT CCT GGA GAA AGT TAC CCG AAA AAA GGA CAA AAT GGA ATC GCA      1344
   Ile Ser Pro Gly Glu Ser Tyr Pro Lys Lys Gly Gln Asn Gly Ile Ala
           435                 440                 445
   ATA ACA TCA ATG GAT GAT TTT AAT TCC CAT CCG ATT ACA TTA AAT AAA      1392
   Ile Thr Ser Met Asp Asp Phe Asn Ser His Pro Ile Thr Leu Asn Lys
       450                 455                 460
   AAA CAA GTA GAT AAT CTG CTA AAT AAT AAA CCT ATG ATG TTG GAA ACA      1440
   Lys Gln Val Asp Asn Leu Leu Asn Asn Lys Pro Met Met Leu Glu Thr
   465                 470                 475                 480
   AAC CAA ACA GAT GGT GTT TAT AAG ATA AAA GAT ACA CAT GGA AAT ATA      1488
   Asn Gln Thr Asp Gly Val Tyr Lys Ile Lys Asp Thr His Gly Asn Ile
                   485                 490                 495
   GTA ACT GGC GGA GAA TGG AAT GGT GTC ATA CAA CAA ATC AAG GCT AAA      1536
   Val Thr Gly Gly Glu Trp Asn Gly Val Ile Gln Gln Ile Lys Ala Lys
               500                 505                 510
   ACA GCG TCT ATT ATT GTG GAT GAT GGG GAA CGT GTA GCA GAA AAA CGT      1584
   Thr Ala Ser Ile Ile Val Asp Asp Gly Glu Arg Val Ala Glu Lys Arg
           515                 520                 525
   GTA GCG GCA AAA GAT TAT GAA AAT CCA GAA GAT AAA ACA CCG TCT TTA      1632
   Val Ala Ala Lys Asp Tyr Glu Asn Pro Glu Asp Lys Thr Pro Ser Leu
       530                 535                 540
   ACT TTA AAA GAT GCC CTG AAG CTT TCA TAT CCA GAT GAA ATA AAA GAA      1680
   Thr Leu Lys Asp Ala Leu Lys Leu Ser Tyr Pro Asp Glu Ile Lys Glu
   545                 550                 555                 560
   ATA GAG GGA TTA TTA TAT TAT AAA AAC AAA CCG ATA TAC GAA TCG AGC      1728
   Ile Glu Gly Leu Leu Tyr Tyr Lys Asn Lys Pro Ile Tyr Glu Ser Ser
                       565                     570                     575
   GTT ATG ACT TAC TTA GAT GAA AAT ACA GCA AAA GAA GTG ACC AAA CAA        1776
   Val Met Thr Tyr Leu Asp Glu Asn Thr Ala Lys Glu Val Thr Lys Gln
               580                     585                     590
   TTA AAT GAT ACC ACT GGG AAA TTT AAA GAT GTA AGT CAT TTA TAT GAT        1824
   Leu Asn Asp Thr Thr Gly Lys Phe Lys Asp Val Ser His Leu Tyr Asp
           595                     600                     605
   GTA AAA CTG ACT CCA AAA ATG AAT GTT ACA ATC AAA TTG TCT ATA CTT        1872
   Val Lys Leu Thr Pro Lys Met Asn Val Thr Ile Lys Leu Ser Ile Leu
       610                     615                     620
   TAT GAT AAT GCT GAG TCT AAT GAT AAC TCA ATT GGT AAA TGG ACA AAC        1920
   Tyr Asp Asn Ala Glu Ser Asn Asp Asn Ser Ile Gly Lys Trp Thr Asn
   625                     630                     635                     640
   ACA AAT ATT GTT TCA GGT GGA AAT AAC GGA AAA AAA CAA TAT TCT TCT        1968
   Thr Asn Ile Val Ser Gly Gly Asn Asn Gly Lys Lys Gln Tyr Ser Ser
                   645                     650                     655
   AAT AAT CCG GAT GCT AAT TTG ACA TTA AAT ACA GAT GCT CAA GAA AAA        2016
   Asn Asn Pro Asp Ala Asn Leu Thr Leu Asn Thr Asp Ala Gln Glu Lys
               660                     665                     670
   TTA AAT AAA AAT CGT GAC TAT TAT ATA AGT TTA TAT ATG AAG TCA GAA        2064
   Leu Asn Lys Asn Arg Asp Tyr Tyr Ile Ser Leu Tyr Met Lys Ser Glu
           675                     680                     685
   AAA AAC ACA CAA TGT GAG ATT ACT ATA GAT GGG GAG ATT TAT CCG ATC        2112
   Lys Asn Thr Gln Cys Glu Ile Thr Ile Asp Gly Glu Ile Tyr Pro Ile
       690                     695                     700
   ACT ACA AAA ACA GTG AAT GTG AAT AAA GAC AAT TAC AAA AGA TTA GAT        2160
   Thr Thr Lys Thr Val Asn Val Asn Lys Asp Asn Tyr Lys Arg Leu Asp
   705                     710                     715                     720
   ATT ATA GCT CAT AAT ATA AAA AGT AAT CCA ATT TCT TCA CTT CAT ATT        2208
   Ile Ile Ala His Asn Ile Lys Ser Asn Pro Ile Ser Ser Leu His Ile
                   725                     730                     735
   AAA ACG AAT GAT GAA ATA ACT TTA TTT TGG GAT GAT ATT TCT ATA ACA        2256
   Lys Thr Asn Asp Glu Ile Thr Leu Phe Trp Asp Asp Ile Ser Ile Thr
               740                     745                     750
   GAT GTA GCA TCA ATA AAA CCG GAA AAT TTA ACA GAT TCA GAA ATT AAA        2304
   Asp Val Ala Ser Ile Lys Pro Glu Asn Leu Thr Asp Ser Glu Ile Lys
           755                     760                     765
   CAG ATT TAT AGT AGG TAT GGT ATT AAG TTA GAA GAT GGA ATC CTT ATT        2352
   Gln Ile Tyr Ser Arg Tyr Gly Ile Lys Leu Glu Asp Gly Ile Leu Ile
       770                     775                     780
   GAT AAA AAA GGT GGG ATT CAT TAT GGT GAA TTT ATT AAT GAA GCT AGT        2400
   Asp Lys Lys Gly Gly Ile His Tyr Gly Glu Phe Ile Asn Glu Ala Ser
   785                     790                     795                     800
   TTT AAT ATT GAA CCA TTG CCA AAT TAT GTG ACC AAA TAT GAA GTT ACT        2448
   Phe Asn Ile Glu Pro Leu Pro Asn Tyr Val Thr Lys Tyr Glu Val Thr
                     805                     810                     815
   TAT AGT AGT GAG TTA GGA CCA AAC GTG AGT GAC ACA CTT GAA AGT GAT      2496
   Tyr Ser Ser Glu Leu Gly Pro Asn Val Ser Asp Thr Leu Glu Ser Asp
               820                     825                     830
   AAA ATT TAC AAG GAT GGG ACA ATT AAA TTT GAT TTT ACC AAA TAT AGT      2544
   Lys Ile Tyr Lys Asp Gly Thr Ile Lys Phe Asp Phe Thr Lys Tyr Ser
           835                     840                     845
   AAA AAT GAA CAA GGA TTA TTT TAT GAC AGT GGA TTA AAT TGG GAC TTT      2592
   Lys Asn Glu Gln Gly Leu Phe Tyr Asp Ser Gly Leu Asn Trp Asp Phe
       850                     855                     860
   AAA ATT AAT GCT ATT ACT TAT GAT GGT AAA GAG ATG AAT GTT TTT CAT      2640
   Lys Ile Asn Ala Ile Thr Tyr Asp Gly Lys Glu Met Asn Val Phe His
   865                 870                     875                 880
   AGA TAT AAT AAA TAG                                                  2655
   Arg Tyr Asn Lys 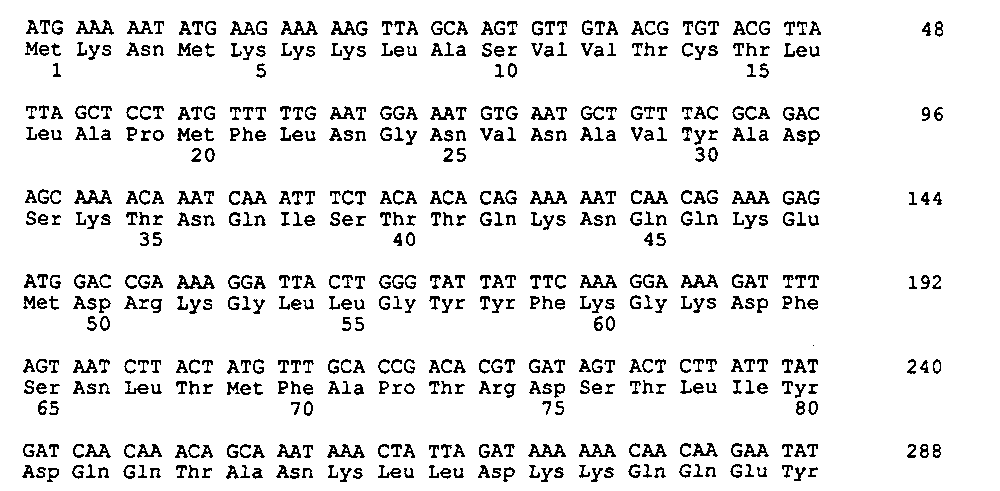
(2) INFORMATION FOR SEQ ID NO: 5:
   (i) SEQUENCE CHARACTERISTICS:
      (A) LENGTH: 884 amino acids
      (B) TYPE: amino acid
      (D) TOPOLOGY: linear
   (ii) MOLECULE TYPE: protein
   (xi) SEQUENCE DESCRIPTION: SEQ ID NO: 5:
   Met Lys Asn Met Lys Lys Lys Leu Ala Ser Val Val Thr Cys Thr Leu
    1               5                  10                  15
   Leu Ala Pro Met Phe Leu Asn Gly Asn Val Asn Ala Val Tyr Ala Asp
               20                  25                  30
   Ser Lys Thr Asn Gln Ile Ser Thr Thr Gln Lys Asn Gln Gln Lys Glu
           35                  40                  45
   Met Asp Arg Lys Gly Leu Leu Gly Tyr Tyr Phe Lys Gly Lys Asp Phe
       50                  55                  60
   Ser Asn Leu Thr Met Phe Ala Pro Thr Arg Asp Ser Thr Leu Ile Tyr
   65                  70                  75                  80
   Asp Gln Gln Thr Ala Asn Lys Leu Leu Asp Lys Lys Gln Gln Glu Tyr
                   85                  90                  95
   Gln Ser Ile Arg Trp Ile Gly Leu Ile Gln Ser Lys Glu Thr Gly Asp
               100                 105                 110
   Phe Thr Phe Asn Leu Ser Glu Asp Glu Gln Ala Ile Ile Glu Ile Asn
           115                 120                 125
   Gly Lys Ile Ile Ser Asn Lys Gly Lys Glu Lys Gln Val Val His Leu
       130                 135                 140
   Glu Lys Gly Lys Leu Val Pro Ile Lys Ile Glu Tyr Gln Ser Asp Thr
   145                 150                 155                 160
   Lys Phe Asn Ile Asp Ser Lys Thr Phe Lys Glu Leu Lys Leu Phe Lys
                   165                 170                 175
   Ile Asp Ser Gln Asn Gln Pro Gln Gln Val Gln Gln Asp Glu Leu Arg
               180                 185                 190
   Asn Pro Glu Phe Asn Lys Lys Glu Ser Gln Glu Phe Leu Ala Lys Pro
           195                 200                 205
   Ser Lys Ile Asn Leu Phe Thr Gln Xaa Met Lys Arg Glu Ile Asp Glu
       210                 215                 220
   Asp Thr Asp Thr Asp Gly Asp Ser Ile Pro Asp Leu Trp Glu Glu Asn
   225                 230                 235                 240
   Gly Tyr Thr Ile Xaa Asn Arg Ile Ala Val Lys Trp Asp Asp Ser Leu
                   245                 250                 255
   Ala Ser Lys Gly Tyr Thr Lys Phe Val Ser Asn Pro Leu Glu Ser His
               260                 265                 270
   Thr Val Gly Asp Pro Tyr Thr Asp Tyr Glu Lys Ala Ala Arg Asp Leu
           275                 280                 285
   Asp Leu Ser Asn Ala Lys Glu Thr Phe Asn Pro Leu Val Ala Ala Phe
       290                 295                 300
   Pro Ser Val Asn Val Ser Met Glu Lys Val Ile Leu Ser Pro Asn Glu
   305                 310                 315                 320
   Asn Leu Ser Asn Ser Val Glu Ser His Ser Ser Thr Asn Trp Ser Tyr
                   325                 330                 335
   Thr Asn Thr Glu Gly Ala Ser Val Glu Ala Gly Ile Gly Pro Lys Gly
               340                 345                 350
   Ile Ser Phe Gly Val Ser Val Asn Tyr Gln His Ser Glu Thr Val Ala
           355                 360                 365
   Gln Glu Trp Gly Thr Ser Thr Gly Asn Thr Ser Gln Phe Asn Thr Ala
       370                 375                 380
   Ser Ala Gly Tyr Leu Asn Ala Asn Val Arg Tyr Asn Asn Val Gly Thr
   385                 390                 395                 400
   Gly Ala Ile Tyr Asp Val Lys Pro Thr Thr Ser Phe Val Leu Asn Asn
                   405                 410                 415
   Asp Thr Ile Ala Thr Ile Thr Ala Lys Ser Asn Ser Thr Ala Leu Asn
               420                 425                 430
   Ile Ser Pro Gly Glu Ser Tyr Pro Lys Lys Gly Gln Asn Gly Ile Ala
           435                 440                 445
   Ile Thr Ser Met Asp Asp Phe Asn Ser His Pro Ile Thr Leu Asn Lys
       450                 455                 460
   Lys Gln Val Asp Asn Leu Leu Asn Asn Lys Pro Met Met Leu Glu Thr
   465                 470                 475                 480
   Asn Gln Thr Asp Gly Val Tyr Lys Ile Lys Asp Thr His Gly Asn Ile
                   485                 490                 495
   Val Thr Gly Gly Glu Trp Asn Gly Val Ile Gln Gln Ile Lys Ala Lys
               500                 505                 510
   Thr Ala Ser Ile Ile Val Asp Asp Gly Glu Arg Val Ala Glu Lys Arg
           515                 520                 525
   Val Ala Ala Lys Asp Tyr Glu Asn Pro Glu Asp Lys Thr Pro Ser Leu
       530                 535                 540
   Thr Leu Lys Asp Ala Leu Lys Leu Ser Tyr Pro Asp Glu Ile Lys Glu
   545                 550                 555                 560
   Ile Glu Gly Leu Leu Tyr Tyr Lys Asn Lys Pro Ile Tyr Glu Ser Ser
                   565                 570                 575
   Val Met Thr Tyr Leu Asp Glu Asn Thr Ala Lys Glu Val Thr Lys Gln
               580                 585                 590
   Leu Asn Asp Thr Thr Gly Lys Phe Lys Asp Val Ser His Leu Tyr Asp
           595                 600                 605
   Val Lys Leu Thr Pro Lys Met Asn Val Thr Ile Lys Leu Ser Ile Leu
       610                 615                 620
   Tyr Asp Asn Ala Glu Ser Asn Asp Asn Ser Ile Gly Lys Trp Thr Asn
   625                 630                 635                 640
   Thr Asn Ile Val Ser Gly Gly Asn Asn Gly Lys Lys Gln Tyr Ser Ser
                   645                 650                 655
   Asn Asn Pro Asp Ala Asn Leu Thr Leu Asn Thr Asp Ala Gln Glu Lys
               660                 665                 670
   Leu Asn Lys Asn Arg Asp Tyr Tyr Ile Ser Leu Tyr Met Lys Ser Glu
           675                 680                 685
   Lys Asn Thr Gln Cys Glu Ile Thr Ile Asp Gly Glu Ile Tyr Pro Ile
       690                 695                 700
   Thr Thr Lys Thr Val Asn Val Asn Lys Asp Asn Tyr Lys Arg Leu Asp
   705                 710                 715                 720
   Ile Ile Ala His Asn Ile Lys Ser Asn Pro Ile Ser Ser Leu His Ile
                   725                 730                 735
   Lys Thr Asn Asp Glu Ile Thr Leu Phe Trp Asp Asp Ile Ser Ile Thr
               740                 745                 750
   Asp Val Ala Ser Ile Lys Pro Glu Asn Leu Thr Asp Ser Glu Ile Lys
           755                 760                 765
   Gln Ile Tyr Ser Arg Tyr Gly Ile Lys Leu Glu Asp Gly Ile Leu Ile
       770                 775                 780
   Asp Lys Lys Gly Gly Ile His Tyr Gly Glu Phe Ile Asn Glu Ala Ser
   785                 790                 795                 800
   Phe Asn Ile Glu Pro Leu Pro Asn Tyr Val Thr Lys Tyr Glu Val Thr
                   805                 810                 815
   Tyr Ser Ser Glu Leu Gly Pro Asn Val Ser Asp Thr Leu Glu Ser Asp
               820                 825                 830
   Lys Ile Tyr Lys Asp Gly Thr Ile Lys Phe Asp Phe Thr Lys Tyr Ser
           835                 840                 845
   Lys Asn Glu Gln Gly Leu Phe Tyr Asp Ser Gly Leu Asn Trp Asp Phe
       850                 855                 860
   Lys Ile Asn Ala Ile Thr Tyr Asp Gly Lys Glu Met Asn Val Phe His
   865                 870                 875                 880
   Arg Tyr Asn Lys
(2) INFORMATION FOR SEQ ID NO: 6:
   (i) SEQUENCE CHARACTERISTICS:
      (A) LENGTH: 2004 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: CDS
      (B) LOCATION: 1..2001
      (C) IDENTIFICATION METHOD: experimental
      (D) OTHER INFORMATION: /product= "80 kDa protein VIP-1" /evidence= EXPERIMENTAL
   (xi) SEQUENCE DESCRIPTION: SEQ ID NO: 6:
   ATG AAA AGG GAA ATT GAT GAA GAC ACG GAT ACG GAT GGG GAC TCT ATT      48
   Met Lys Arg Glu Ile Asp Glu Asp Thr Asp Thr Asp Gly Asp Ser Ile
     1               5                  10                  15
   CCT GAC CTT TGG GAA GAA AAT GGG TAT ACG ATT CAM AAT AGA ATC GCT      96
   Pro Asp Leu Trp Glu Glu Asn Gly Tyr Thr Ile Xaa Asn Arg Ile Ala
                20                  25                  30
   GTA AAG TGG GAC GAT TCT CTA GCA AGT AAA GGG TAT ACG AAA TTT GTT     144
   Val Lys Trp Asp Asp Ser Leu Ala Ser Lys Gly Tyr Thr Lys Phe Val
            35                  40                  45
   TCA AAT CCA CTA GAA AGT CAC ACA GTT GGT GAT CCT TAT ACA GAT TAT      192
   Ser Asn Pro Leu Glu Ser His Thr Val Gly Asp Pro Tyr Thr Asp Tyr
        50                  55                  60
   GAA AAG GCA GCA AGA GAT CTA GAT TTG TCA AAT GCA AAG GAA ACG TTT      240
   Glu Lys Ala Ala Arg Asp Leu Asp Leu Ser Asn Ala Lys Glu Thr Phe
    65                  70                  75                  80
   AAC CCA TTG GTA GCT GCT TTT CCA AGT GTG AAT GTT AGT ATG GAA AAG      288
   Asn Pro Leu Val Ala Ala Phe Pro Ser Val Asn Val Ser Met Glu Lys
                    85                  90                  95
   GTG ATA TTA TCA CCA AAT GAA AAT TTA TCC AAT AGT GTA GAG TCT CAT      336
   Val Ile Leu Ser Pro Asn Glu Asn Leu Ser Asn Ser Val Glu Ser His
               100                 105                 110
   TCA TCC ACG AAT TGG TCT TAT ACA AAT ACA GAA GGT GCT TCT GTT GAA      384
   Ser Ser Thr Asn Trp Ser Tyr Thr Asn Thr Glu Gly Ala Ser Val Glu
           115                 120                 125
   GCG GGG ATT GGA CCA AAA GGT ATT TCG TTC GGA GTT AGC GTA AAC TAT      432
   Ala Gly Ile Gly Pro Lys Gly Ile Ser Phe Gly Val Ser Val Asn Tyr
       130                 135                 140
   CAA CAC TCT GAA ACA GTT GCA CAA GAA TGG GGA ACA TCT ACA GGA AAT      480
   Gln His Ser Glu Thr Val Ala Gln Glu Trp Gly Thr Ser Thr Gly Asn
   145                 150                 155                 160
   ACT TCG CAA TTC AAT ACG GCT TCA GCG GGA TAT TTA AAT GCA AAT GTT      528
   Thr Ser Gln Phe Asn Thr Ala Ser Ala Gly Tyr Leu Asn Ala Asn Val
                   165                 170                 175
   CGA TAT AAC AAT GTA GGA ACT GGT GCC ATC TAC GAT GTA AAA CCT ACA      576
   Arg Tyr Asn Asn Val Gly Thr Gly Ala Ile Tyr Asp Val Lys Pro Thr
               180                 185                 190
   ACA AGT TTT GTA TTA AAT AAC GAT ACT ATC GCA ACT ATT ACG GCG AAA      624
   Thr Ser Phe Val Leu Asn Asn Asp Thr Ile Ala Thr Ile Thr Ala Lys
           195                 200                 205
   TCT AAT TCT ACA GCC TTA AAT ATA TCT CCT GGA GAA AGT TAC CCG AAA      672
   Ser Asn Ser Thr Ala Leu Asn Ile Ser Pro Gly Glu Ser Tyr Pro Lys
       210                 215                 220
   AAA GGA CAA AAT GGA ATC GCA ATA ACA TCA ATG GAT GAT TTT AAT TCC      720
   Lys Gly Gln Asn Gly Ile Ala Ile Thr Ser Met Asp Asp Phe Asn Ser
   225                 230                 235                 240
   CAT CCG ATT ACA TTA AAT AAA AAA CAA GTA GAT AAT CTG CTA AAT AAT      768
   His Pro Ile Thr Leu Asn Lys Lys Gln Val Asp Asn Leu Leu Asn Asn
                   245                 250                 255
   AAA CCT ATG ATG TTG GAA ACA AAC CAA ACA GAT GGT GTT TAT AAG ATA      816
   Lys Pro Met Met Leu Glu Thr Asn Gln Thr Asp Gly Val Tyr Lys Ile
               260                 265                 270
   AAA GAT ACA CAT GGA AAT ATA GTA ACT GGC GGA GAA TGG AAT GGT GTC      864
   Lys Asp Thr His Gly Asn Ile Val Thr Gly Gly Glu Trp Asn Gly Val
           275                     280                     285
   ATA CAA CAA ATC AAG GCT AAA ACA GCG TCT ATT ATT GTG GAT GAT GGG        912
   Ile Gln Gln Ile Lys Ala Lys Thr Ala Ser Ile Ile Val Asp Asp Gly
       290                     295                     300
   GAA CGT GTA GCA GAA AAA CGT GTA GCG GCA AAA GAT TAT GAA AAT CCA        960
   Glu Arg Val Ala Glu Lys Arg Val Ala Ala Lys Asp Tyr Glu Asn Pro
   305                     310                     315                     320
   GAA GAT AAA ACA CCG TCT TTA ACT TTA AAA GAT GCC CTG AAG CTT TCA       1008
   Glu Asp Lys Thr Pro Ser Leu Thr Leu Lys Asp Ala Leu Lys Leu Ser
                   325                     330                     335
   TAT CCA GAT GAA ATA AAA GAA ATA GAG GGA TTA TTA TAT TAT AAA AAC       1056
   Tyr Pro Asp Glu Ile Lys Glu Ile Glu Gly Leu Leu Tyr Tyr Lys Asn
               340                     345                     350
   AAA CCG ATA TAC GAA TCG AGC GTT ATG ACT TAC TTA GAT GAA AAT ACA       1104
   Lys Pro Ile Tyr Glu Ser Ser Val Met Thr Tyr Leu Asp Glu Asn Thr
           355                     360                     365
   GCA AAA GAA GTG ACC AAA CAA TTA AAT GAT ACC ACT GGG AAA TTT AAA       1152
   Ala Lys Glu Val Thr Lys Gln Leu Asn Asp Thr Thr Gly Lys Phe Lys
       370                     375                     380
   GAT GTA AGT CAT TTA TAT GAT GTA AAA CTG ACT CCA AAA ATG AAT GTT       1200
   Asp Val Ser His Leu Tyr Asp Val Lys Leu Thr Pro Lys Met Asn Val
   385                     390                     395                     400
   ACA ATC AAA TTG TCT ATA CTT TAT GAT AAT GCT GAG TCT AAT GAT AAC       1248
   Thr Ile Lys Leu Ser Ile Leu Tyr Asp Asn Ala Glu Ser Asn Asp Asn
                   405                     410                     415
   TCA ATT GGT AAA TGG ACA AAC ACA AAT ATT GTT TCA GGT GGA AAT AAC       1296
   Ser Ile Gly Lys Trp Thr Asn Thr Asn Ile Val Ser Gly Gly Asn Asn
               420                     425                     430
   GGA AAA AAA CAA TAT TCT TCT AAT AAT CCG GAT GCT AAT TTG ACA TTA       1344
   Gly Lys Lys Gln Tyr Ser Ser Asn Asn Pro Asp Ala Asn Leu Thr Leu
           435                     440                     445
   AAT ACA GAT GCT CAA GAA AAA TTA AAT AAA AAT CGT GAC TAT TAT ATA       1392
   Asn Thr Asp Ala Gln Glu Lys Leu Asn Lys Asn Arg Asp Tyr Tyr Ile
       450                     455                     460
   AGT TTA TAT ATG AAG TCA GAA AAA AAC ACA CAA TGT GAG ATT ACT ATA       1440
   Ser Leu Tyr Met Lys Ser Glu Lys Asn Thr Gln Cys Glu Ile Thr Ile
   465                     470                     475                     480
   GAT GGG GAG ATT TAT CCG ATC ACT ACA AAA ACA GTG AAT GTG AAT AAA       1488
   Asp Gly Glu Ile Tyr Pro Ile Thr Thr Lys Thr Val Asn Val Asn Lys
                   485                     490                     495
   GAC AAT TAC AAA AGA TTA GAT ATT ATA GCT CAT AAT ATA AAA AGT AAT       1536
   Asp Asn Tyr Lys Arg Leu Asp Ile Ile Ala His Asn Ile Lys Ser Asn
               500                     505                     510
   CCA ATT TCT TCA CTT CAT ATT AAA ACG AAT GAT GAA ATA ACT TTA TTT       1584
   Pro Ile Ser Ser Leu His Ile Lys Thr Asn Asp Glu Ile Thr Leu Phe
           515                 520                 525
   TGG GAT GAT ATT TCT ATA ACA GAT GTA GCA TCA ATA AAA CCG GAA AAT      1632
   Trp Asp Asp Ile Ser Ile Thr Asp Val Ala Ser Ile Lys Pro Glu Asn
       530                 535                 540
   TTA ACA GAT TCA GAA ATT AAA CAG ATT TAT AGT AGG TAT GGT ATT AAG      1680
   Leu Thr Asp Ser Glu Ile Lys Gln Ile Tyr Ser Arg Tyr Gly Ile Lys
   545                 550                 555                 560
   TTA GAA GAT GGA ATC CTT ATT GAT AAA AAA GGT GGG ATT CAT TAT GGT      1728
   Leu Glu Asp Gly Ile Leu Ile Asp Lys Lys Gly Gly Ile His Tyr Gly
                   565                 570                 575
   GAA TTT ATT AAT GAA GCT AGT TTT AAT ATT GAA CCA TTG CCA AAT TAT      1776
   Glu Phe Ile Asn Glu Ala Ser Phe Asn Ile Glu Pro Leu Pro Asn Tyr
               580                 585                 590
   GTG ACC AAA TAT GAA GTT ACT TAT AGT AGT GAG TTA GGA CCA AAC GTG      1824
   Val Thr Lys Tyr Glu Val Thr Tyr Ser Ser Glu Leu Gly Pro Asn Val
           595                 600                 605
   AGT GAC ACA CTT GAA AGT GAT AAA ATT TAC AAG GAT GGG ACA ATT AAA      1872
   Ser Asp Thr Leu Glu Ser Asp Lys Ile Tyr Lys Asp Gly Thr Ile Lys
       610                 615                 620
   TTT GAT TTT ACC AAA TAT AGT AAA AAT GAA CAA GGA TTA TTT TAT GAC      1920
   Phe Asp Phe Thr Lys Tyr Ser Lys Asn Glu Gln Gly Leu Phe Tyr Asp
   625                 630                 635                 640
   AGT GGA TTA AAT TGG GAC TTT AAA ATT AAT GCT ATT ACT TAT GAT GGT      1968
   Ser Gly Leu Asn Trp Asp Phe Lys Ile Asn Ala Ile Thr Tyr Asp Gly
                   645                 650                 655
   AAA GAG ATG AAT GTT TTT CAT AGA TAT AAT AAA TAG                      2004
   Lys Glu Met Asn Val Phe His Arg Tyr Asn Lys
               660                 665
(2) INFORMATION FOR SEQ ID NO: 7:
   (i) SEQUENCE CHARACTERISTICS:
      (A) LENGTH: 667 amino acids
      (B) TYPE: amino acid
      (D) TOPOLOGY: linear
   (ii) MOLECULE TYPE: protein
   (xi) SEQUENCE DESCRIPTION: SEQ ID NO: 7:
   Met Lys Arg Glu Ile Asp Glu Asp Thr Asp Thr Asp Gly Asp Ser Ile
    1               5                  10                  15
   Pro Asp Leu Trp Glu Glu Asn Gly Tyr Thr Ile Xaa Asn Arg Ile Ala
               20                  25                  30
   Val Lys Trp Asp Asp Ser Leu Ala Ser Lys Gly Tyr Thr Lys Phe Val
           35                  40                  45
   Ser Asn Pro Leu Glu Ser His Thr Val Gly Asp Pro Tyr Thr Asp Tyr
        50                  55                  60
   Glu Lys Ala Ala Arg Asp Leu Asp Leu Ser Asn Ala Lys Glu Thr Phe
    65                  70                  75                  80
   Asn Pro Leu Val Ala Ala Phe Pro Ser Val Asn Val Ser Met Glu Lys
                   85                  90                  95
   Val Ile Leu Ser Pro Asn Glu Asn Leu Ser Asn Ser Val Glu Ser His
               100                 105                 110
   Ser Ser Thr Asn Trp Ser Tyr Thr Asn Thr Glu Gly Ala Ser Val Glu
           115                 120                 125
   Ala Gly Ile Gly Pro Lys Gly Ile Ser Phe Gly Val Ser Val Asn Tyr
       130                 135                 140
   Gln His Ser Glu Thr Val Ala Gln Glu Trp Gly Thr Ser Thr Gly Asn
   145                 150                 155                 160
   Thr Ser Gln Phe Asn Thr Ala Ser Ala Gly Tyr Leu Asn Ala Asn Val
                   165                 170                 175
   Arg Tyr Asn Asn Val Gly Thr Gly Ala Ile Tyr Asp Val Lys Pro Thr
               180                 185                 190
   Thr Ser Phe Val Leu Asn Asn Asp Thr Ile Ala Thr Ile Thr Ala Lys
           195                 200                 205
   Ser Asn Ser Thr Ala Leu Asn Ile Ser Pro Gly Glu Ser Tyr Pro Lys
       210                 215                 220
   Lys Gly Gln Asn Gly Ile Ala Ile Thr Ser Met Asp Asp Phe Asn Ser
   225                 230                 235                 240
   His Pro Ile Thr Leu Asn Lys Lys Gln Val Asp Asn Leu Leu Asn Asn
                   245                 250                 255
   Lys Pro Met Met Leu Glu Thr Asn Gln Thr Asp Gly Val Tyr Lys Ile
               260                 265                 270
   Lys Asp Thr His Gly Asn Ile Val Thr Gly Gly Glu Trp Asn Gly Val
           275                 280                 285
   Ile Gln Gln Ile Lys Ala Lys Thr Ala Ser Ile Ile Val Asp Asp Gly
       290                 295                 300
   Glu Arg Val Ala Glu Lys Arg Val Ala Ala Lys Asp Tyr Glu Asn Pro
   305                 310                 315                 320
   Glu Asp Lys Thr Pro Ser Leu Thr Leu Lys Asp Ala Leu Lys Leu Ser
                   325                 330                 335
   Tyr Pro Asp Glu Ile Lys Glu Ile Glu Gly Leu Leu Tyr Tyr Lys Asn
               340                 345                 350
   Lys Pro Ile Tyr Glu Ser Ser Val Met Thr Tyr Leu Asp Glu Asn Thr
           355                 360                 365
   Ala Lys Glu Val Thr Lys Gln Leu Asn Asp Thr Thr Gly Lys Phe Lys
       370                 375                 380
   Asp Val Ser His Leu Tyr Asp Val Lys Leu Thr Pro Lys Met Asn Val
   385                 390                 395                 400
   Thr Ile Lys Leu Ser Ile Leu Tyr Asp Asn Ala Glu Ser Asn Asp Asn
                   405                 410                 415
   Ser Ile Gly Lys Trp Thr Asn Thr Asn Ile Val Ser Gly Gly Asn Asn
               420                 425                 430
   Gly Lys Lys Gln Tyr Ser Ser Asn Asn Pro Asp Ala Asn Leu Thr Leu
           435                 440                 445
   Asn Thr Asp Ala Gln Glu Lys Leu Asn Lys Asn Arg Asp Tyr Tyr Ile
       450                 455                 460
   Ser Leu Tyr Met Lys Ser Glu Lys Asn Thr Gln Cys Glu Ile Thr Ile
   465                 470                 475                 480
   Asp Gly Glu Ile Tyr Pro Ile Thr Thr Lys Thr Val Asn Val Asn Lys
                   485                 490                 495
   Asp Asn Tyr Lys Arg Leu Asp Ile Ile Ala His Asn Ile Lys Ser Asn
               500                 505                 510
   Pro Ile Ser Ser Leu His Ile Lys Thr Asn Asp Glu Ile Thr Leu Phe
           515                 520                 525
   Trp Asp Asp Ile Ser Ile Thr Asp Val Ala Ser Ile Lys Pro Glu Asn
       530                 535                 540
   Leu Thr Asp Ser Glu Ile Lys Gln Ile Tyr Ser Arg Tyr Gly Ile Lys
   545                 550                 555                 560
   Leu Glu Asp Gly Ile Leu Ile Asp Lys Lys Gly Gly Ile His Tyr Gly
                   565                 570                 575
   Glu Phe Ile Asn Glu Ala Ser Phe Asn Ile Glu Pro Leu Pro Asn Tyr
               580                 585                 590
   Val Thr Lys Tyr Glu Val Thr Tyr Ser Ser Glu Leu Gly Pro Asn Val
           595                 600                 605
   Ser Asp Thr Leu Glu Ser Asp Lys Ile Tyr Lys Asp Gly Thr Ile Lys
       610                 615                 620
   Phe Asp Phe Thr Lys Tyr Ser Lys Asn Glu Gln Gly Leu Phe Tyr Asp
   625                 630                 635                 640
   Ser Gly Leu Asn Trp Asp Phe Lys Ile Asn Ala Ile Thr Tyr Asp Gly
                   645                 650                 655
   Lys Glu Met Asn Val Phe His Arg Tyr Asn Lys
               660                 665
(2) INFORMATION FOR SEQ ID NO: 8:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus cereus
      (B) STRAIN: AB78
      (C) INDIVIDUAL ISOLATE: NRRL B-21058
   (ix) FEATURE:
      (A) NAME/KEY: Peptide
      (B) LOCATION: 1..16
      (D) OTHER INFORMATION: /note= "N-terminal sequence of protein purified from strain AB78"
   (xi) SEQUENCE DESCRIPTION: SEQ ID NO: 8:
   Lys Arg Glu Ile Asp Glu Asp Thr Asp Thr Asx Gly Asp Ser Ile Pro
   1               5                   10                  15
(2) INFORMATION FOR SEQ ID NO: 9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (ix) FEATURE:
      (A) NAME/KEY: misc_feature
      (B) LOCATION: 1..21
      (D) OTHER INFORMATION: /note= "Oligonucleotide probe based on amino acids 3 to 9 of SEQ ID NO:8, using codon usage of Bacillus thuringiensis"
   (xi) SEQUENCE DESCRIPTION: SEQ ID NO: 9:
   GAAATTGATC AAGATACNGA T                                             21
(2) INFORMATION FOR SEQ ID NO: 10:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
      (B) STRAIN: AB88
   (ix) FEATURE:
      (A) NAME/KEY: Peptide
      (B) LOCATION: 1..14
      (D) OTHER INFORMATION: /note= "N-terminal amino acid sequence of protein known as anion exchange fraction 23 (smaller)"
   (xi) SEQUENCE DESCRIPTION: SEQ ID NO: 10:
   Xaa Glu Pro Phe Val Ser Ala Xaa Xaa Xaa Gln Xaa Xaa Xaa
   1               5                   10
(2) INFORMATION FOR SEQ ID NO: 11:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
      (B) STRAIN: AB88
   (ix) FEATURE:
      (A) NAME/KEY: Peptide
      (B) LOCATION: 1..13
      (D) OTHER INFORMATION: /note= "N-terminal amino acid sequence of protein known as anion exchange fraction 23 (larger)"
   (xi) SEQUENCE DESCRIPTION: SEQ ID NO: 11:
   Xaa Glu Tyr Glu Asn Val Glu Pro Phe Val Ser Ala Xaa
   1               5                   10
(2) INFORMATION FOR SEQ ID NO: 12:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thurigiensis
      (B) STRAIN: AB88
   (ix) FEATURE:
      (A) NAME/KEY: Peptide
      (B) LOCATION: 1..14
      (D) OTHER INFORMATION: /note= "N-terminal sequence of 80 kDa VIP active against Agrotis ipsilon"
   (xi) SEQUENCE DESCRIPTION: SEQ ID NO: 12:
   Met Asn Lys Asn Asn Thr Lys Leu Pro Thr Arg Ala Leu Pro
   1               5                   10
(2) INFORMATION FOR SEQ ID NO: 13:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
      (B) STRAIN: AB88
   (ix) FEATURE:
      (A) NAME/KEY: Peptide
      (B) LOCATION: 1..15
      (D) OTHER INFORMATION: /note= "N-terminal amino acid sequence of 35 kDa VIP active against Agrotis ipsilon"
   (xi) SEQUENCE DESCRIPTION: SEQ ID NO: 13:
   Ala Leu Ser Glu Asn Thr Gly Lys Asp Gly Gly Tyr Ile Val Pro
   1               5                   10                  15
(2) INFORMATION FOR SEQ ID NO: 14:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
   (ix) FEATURE:
      (A) NAME/KEY: Peptide
      (B) LOCATION: 1..9
      (D) OTHER INFORMATION: /note= "N-terminal sequence of a 130 kDa delta-endotoxin"
   (xi) SEQUENCE DESCRIPTION: SEQ ID NO: 14:
   Met Asp Asn Asn Pro Asn Ile Asn Glu
   1               5
(2) INFORMATION FOR SEQ ID NO: 15:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (ix) FEATURE:
      (A) NAME/KEY: Peptide
      (B) LOCATION: 1..9
      (D) OTHER INFORMATION: /note= "N-terminal sequence of 80 kDa delta-endotoxin"
   (xi) SEQUENCE DESCRIPTION: SEQ ID NO: 15:
   Met Asp Asn Asn Pro Asn Ile Asn Glu
   1               5
(2) INFORMATION FOR SEQ ID NO: 16:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (v) FRAGMENT TYPE: N-terminal
   (vi) ORIGINAL SOURCE:
      (A) ORGANISM: Bacillus thuringiensis
   (ix) FEATURE:
      (A) NAME/KEY: Peptide
      (B) LOCATION: 1..11
      (D) OTHER INFORMATION: /note= "N-terminal sequence from 60 kDa delta-endotoxin"
   (xi) SEQUENCE DESCRIPTION: SEQ ID NO: 16:
   Met Asn Val Leu Asn Ser Gly Arg Thr Thr Ile
   1               5                   10
(2) INFORMATION FOR SEQ ID NO: 17:
   (i) SEQUENCE CHARACTERISTICS:
      (A) LENGTH: 2655 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 17:
   ATGAAGAACA TGAAGAAGAA GCTGGCCAGC GTGGTGACCT GCACCCTGCT GGCCCCCATG 60
   TTCCTGAACG GCAACGTGAA CGCCGTGTAC GCCGACAGCA AGACCAACCA GATCAGCACC 120
   ACCCAGAAGA ACCAGCAGAA GGAGATGGAC CGCAAGGGCC TGCTGGGCTA CTACTTCAAG 180
   GGCAAGGACT TCAGCAACCT GACCATGTTC GCCCCCACGC GTGACAGCAC CCTGATCTAC 240
   GACCAGCAGA CCGCCAACAA GCTGCTGGAC AAGAAGCAGC AGGAGTACCA GAGCATCCGC 300
   TGGATCGGCC TGATCCAGAG CAAGGAGACC GGCGACTTCA CCTTCAACCT GAGCGAGGAC 360
   GAGCAGGCCA TCATCGAGAT CAACGGCAAG ATCATCAGCA ACAAGGGCAA GGAGAAGCAG 420
   GTGGTGCACC TGGAGAAGGG CAAGCTGGTG CCCATCAAGA TCGAGTACCA GAGCGACACC 480
   AAGTTCAACA TCGACAGCAA GACCTTCAAG GAGCTGAAGC TTTTCAAGAT CGACAGCCAG 540
   AACCAGCCCC AGCAGGTGCA GCAGGACGAG CTGCGCAACC CCGAGTTCAA CAAGAAGGAG 600
   AGCCAGGAGT TCCTGGCCAA GCCCAGCAAG ATCAACCTGT TCACCCAGCA GATGAAGCGC 660
   GAGATCGACG AGGACACCGA CACCGACGGC GACAGCATCC CCGACCTGTG GGAGGAGAAC 720
   GGCTACACCA TCCAGAACCG CATCGCCGTG AAGTGGGACG ACAGCCTGGC TAGCAAGGGC 780
   TACACCAAGT TCGTGAGCAA CCCCCTGGAG AGCCACACCG TGGGCGACCC CTACACCGAC 840
   TACGAGAAGG CCGCCCGCGA CCTGGACCTG AGCAACGCCA AGGAGACCTT CAACCCCCTG 900
   GTGGCCGCCT TCCCCAGCGT GAACGTGAGC ATGGAGAAGG TGATCCTGAG CCCCAACGAG 960
   AACCTGAGCA ACAGCGTGGA GAGCCACTCG AGCACCAACT GGAGCTACAC CAACACCGAG 1020
   GGCGCCAGCG TGGAGGCCGG CATCGGTCCC AAGGGCATCA GCTTCGGCGT GAGCGTGAAC 1080
   TACCAGCACA GCGAGACCGT GGCCCAGGAG TGGGGCACCA GCACCGGCAA CACCAGCCAG 1140
   TTCAACACCG CCAGCGCCGG CTACCTGAAC GCCAACGTGC GCTACAACAA CGTGGGCACC 1200
   GGCGCCATCT ACGACGTGAA GCCCACCACC AGCTTCGTGC TGAACAACGA CACCATCGCC 1260
   ACCATCACCG CCAAGTCGAA TTCCACCGCC CTGAACATCA GCCCCGGCGA GAGCTACCCC 1320
   AAGAAGGGCC AGAACGGCAT CGCCATCACC AGCATGGACG ACTTCAACAG CCACCCCATC 1380
   ACCCTGAACA AGAAGCAGGT GGACAACCTG CTGAACAACA AGCCCATGAT GCTGGAGACC 1440
   AACCAGACCG ACGGCGTCTA CAAGATCAAG GACACCCACG GCAACATCGT GACCGGCGGC 1500
   GAGTGGAACG GCGTGATCCA GCAGATCAAG GCCAAGACCG CCAGCATCAT CGTCGACGAC 1560
   GGCGAGCGCG TGGCCGAGAA GCGCGTGGCC GCCAAGGACT ACGAGAACCC CGAGGACAAG 1620
   ACCCCCAGCC TGACCCTGAA GGACGCCCTG AAGCTGAGCT ACCCCGACGA GATCAAGGAG 1680
   ATCGAGGGCC TGCTGTACTA CAAGAACAAG CCCATCTACG AGAGCAGCGT GATGACCTAT 1740
   CTAGACGAGA ACACCGCCAA GGAGGTGACC AAGCAGCTGA ACGACACCAC CGGCAAGTTC 1800
   AAGGACGTGA GCCACCTGTA CGACGTGAAG CTGACCCCCA AGATGAACGT GACCATCAAG 1860
   CTGAGCATCC TGTACGACAA CGCCGAGAGC AACGACAACA GCATCGGCAA GTGGACCAAC 1920
   ACCAACATCG TGAGCGGCGG CAACAACGGC AAGAAGCAGT ACAGCAGCAA CAACCCCGAC 1980
   GCCAACCTGA CCCTGAACAC CGACGCCCAG GAGAAGCTGA ACAAGAACCG CGACTACTAC 2040
   ATCAGCCTGT ACATGAAGAG CGAGAAGAAC ACCCAGTGCG AGATCACCAT CGACGGCGAG 2100
   ATATACCCCA TCACCACCAA GACCGTGAAC GTGAACAAGG ACAACTACAA GCGCCTGGAC 2160
   ATCATCGCCC ACAACATCAA GAGCAACCCC ATCAGCAGCC TGCACATCAA GACCAACGAC 2220
   GAGATCACCC TGTTCTGGGA CGACATATCG ATTACCGACG TCGCCAGCAT CAAGCCCGAG 2280
   AACCTGACCG ACAGCGAGAT CAAGCAGATA TACAGTCGCT ACGGCATCAA GCTGGAGGAC 2340
   GGCATCCTGA TCGACAAGAA GGGCGGCATC CACTACGGCG AGTTCATCAA CGAGGCCAGC 2400
   TTCAACATCG AGCCCCTGCA GAACTACGTG ACCAAGTACG AGGTGACCTA CAGCAGCGAG 2460
   CTGGGCCCCA ACGTGAGCGA CACCCTGGAG AGCGACAAGA TTTACAAGGA CGGCACCATC 2520
   AAGTTCGACT TCACCAAGTA CAGCAAGAAC GAGCAGGGCC TGTTCTACGA CAGCGGCCTG 2580
   AACTGGGACT TCAAGATCAA CGCCATCACC TACGACGGCA AGGAGATGAA CGTGTTCCAC 2640
   CGCTACAACA AGTAG 2655
(2) INFORMATION FOR SEQ ID NO: 18:
   (i) SEQUENCE CHARACTERISTICS:
      (A) LENGTH: 2010 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (xi) SEQUENCE DESCRIPTION: SEQ ID NO: 18:
   GGATCCATGA AGCGCGAGAT CGACGAGGAC ACCGACACCG ACGGCGACAG CATCCCCGAC 60
   CTGTGGGAGG AGAACGGCTA CACCATCCAG AACCGCATCG CCGTGAAGTG GGACGACAGC 120
   CTGGCTAGCA AGGGCTACAC CAAGTTCGTG AGCAACCCCC TGGAGAGCCA CACCGTGGGC 180
   GACCCCTACA CCGACTACGA GAAGGCCGCC CGCGACCTGG ACCTGAGCAA CGCCAAGGAG 240
   ACCTTCAACC CCCTGGTGGC CGCCTTCCCC AGCGTGAACG TGAGCATGGA GAAGGTGATC 300
   CTGAGCCCCA ACGAGAACCT GAGCAACAGC GTGGAGAGCC ACTCGAGCAC CAACTGGAGC 360
   TACACCAACA CCGAGGGCGC CAGCGTGGAG GCCGGCATCG GTCCCAAGGG CATCAGCTTC 420
   GGCGTGAGCG TGAACTACCA GCACAGCGAG ACCGTGGCCC AGGAGTGGGG CACCAGCACC 480
   GGCAACACCA GCCAGTTCAA CACCGCCAGC GCCGGCTACC TGAACGCCAA CGTGCGCTAC 540
   AACAACGTGG GCACCGGCGC CATCTACGAC GTGAAGCCCA CCACCAGCTT CGTGCTGAAC 600
   AACGACACCA TCGCCACCAT CACCGCCAAG TCGAATTCCA CCGCCCTGAA CATCAGCCCC 660
   GGCGAGAGCT ACCCCAAGAA GGGCCAGAAC GGCATCGCCA TCACCAGCAT GGACGACTTC 720
   AACAGCCACC CCATCACCCT GAACAAGAAG CAGGTGGACA ACCTGCTGAA CAACAAGCCC 780
   ATGATGCTGG AGACCAACCA GACCGACGGC GTCTACAAGA TCAAGGACAC CCACGGCAAC 840
   ATCGTGACCG GCGGCGAGTG GAACGGCGTG ATCCAGCAGA TCAAGGCCAA GACCGCCAGC 900
   ATCATCGTCG ACGACGGCGA GCGCGTGGCC GAGAAGCGCG TGGCCGCCAA GGACTACGAG 960
   AACCCCGAGG ACAAGACCCC CAGCCTGACC CTGAAGGACG CCCTGAAGCT GAGCTACCCC 1020
   GACGAGATCA AGGAGATCGA GGGCCTGCTG TACTACAAGA ACAAGCCCAT CTACGAGAGC 1080
   AGCGTGATGA CCTATCTAGA CGAGAACACC GCCAAGGAGG TGACCAAGCA GCTGAACGAC 1140
   ACCACCGGCA AGTTCAAGGA CGTGAGCCAC CTGTACGACG TGAAGCTGAC CCCCAAGATG 1200
   AACGTGACCA TCAAGCTGAG CATCCTGTAC GACAACGCCG AGAGCAACGA CAACAGCATC 1260
   GGCAAGTGGA CCAACACCAA CATCGTGAGC GGCGGCAACA ACGGCAAGAA GCAGTACAGC 1320
   AGCAACAACC CCGACGCCAA CCTGACCCTG AACACCGACG CCCAGGAGAA GCTGAACAAG 1380
   AACCGCGACT ACTACATCAG CCTGTACATG AAGAGCGAGA AGAACACCCA GTGCGAGATC 1440
   ACCATCGACG GCGAGATATA CCCCATCACC ACCAAGACCG TGAACGTGAA CAAGGACAAC 1500
   TACAAGCGCC TGGACATCAT CGCCCACAAC ATCAAGAGCA ACCCCATCAG CAGCCTGCAC 1560
   ATCAAGACCA ACGACGAGAT CACCCTGTTC TGGGACGACA TATCGATTAC CGACGTCGCC 1620
   AGCATCAAGC CCGAGAACCT GACCGACAGC GAGATCAAGC AGATATACAG TCGCTACGGC 1680
   ATCAAGCTGG AGGACGGCAT CCTGATCGAC AAGAAGGGCG GCATCCACTA CGGCGAGTTC 1740
   ATCAACGAGG CCAGCTTCAA CATCGAGCCC CTGCAGAACT ACGTGACCAA GTACGAGGTG 1800
   ACCTACAGCA GCGAGCTGGG CCCCAACGTG AGCGACACCC TGGAGAGCGA CAAGATTTAC 1860
   AAGGACGGCA CCATCAAGTT CGACTTCACC AAGTACAGCA AGAACGAGCA GGGCCTGTTC 1920
   TACGACAGCG GCCTGAACTG GGACTTCAAG ATCAACGCCA TCACCTACGA CGGCAAGGAG 1980
   ATGAACGTGT TCCACCGCTA CAACAAGTAG                                    2010

## Claims

1. A *Bacillus* strain selected from the group consisting of the strains with accession numbers B-21058, B21060, NRRL B-21225, NRRL B-21226, NRRL B-21227, NRRL B-21228, NRRL B-21229 and NRRL B-21230, wherein said strain produces a pesticidal protein during vegetative growth that is isolatable from liquid culture media, wherein said protein is encoded by a nucleotide sequence that hybridizes to a nucleotide sequence of SEQ ID NO: 1, 4, 6, 9, 17 or 18, or wherein said protein cross-reacts with antibodies raised against the proteins of SEQ ID NO: 2, 3, 5, 7, 8, or 10-16.

2. A *Bacillus* strain according to claim 1, wherein said protein is capable of killing pests selected from insects, fungi, bacteria, nematodes, mites, ticks, protozoan pathogens, and animal parasites.

3. A *Bacillus* strain according to claim 2, wherein said protein is capable of killing insects selected from orders Coleoptera, Diptera, Hymenoptera, Lepidoptera, Homoptera, Hemiptera, Orthroptera, Thysanoptera, Dermaptera, Isoptera, Mallophaga, Anoplura, Siphonaptera, or Trichoptera.

4. A *Bacillus* strain according to claim 3, wherein said coleopteran species is a *Diabrotica.*

5. A *Bacillus* strain according to claim 4, wherein said *Diabrotica* is *Diabrotica virgifera virgifera* or *Diabrotica longicornis barberi*.

6. A *Bacillus* strain according to claim 3, wherein said lepidopteran species is an *Agrotis.*

7. A *Bacillus* strain according to claim 6, wherein said *Agrotis* is *Agrotis ipsilon.*

8. A *Bacillus* strain according to claim 1, wherein said protein has a molecular weight of 30 kd or greater.

9. A *Bacillus* strain according to claim 8, wherein said protein has a molecular weight of about 60 to about 100 kd.

10. A *Bacillus* strain according to claim 9, wherein said protein has a molecular weight of about 80 kd.

11. A *Bacillus* strain according to claim 10, wherein said protein has the sequence given in SEQ ID NO:7.

12. A *Bacillus* strain according to claim 9, wherein said protein has a molecular weight of about 100 kDa.

13. A *Bacillus* strain according to claim 12, wherein said protein has the sequence given in SEQ ID NO:5.

14. A pesticidal protein isolatable during the vegetative growth phase *of Bacillus* spp. or active fragments thereof, wherein said protein is isolatable from liquid culture media, and wherein said protein is encoded by a nucleotide sequence that hybridizes to a nucleotide sequence of SEQ ID NO: 1, 4, 6, 9, 17 or 18, or wherein said protein cross-reacts with antibodies raised against the proteins of SEQ ID NO: 2, 3, 5, 7, 8, or 10 to 16.

15. A pesticidal protein according to claim 14 which is a multimeric protein.

16. A pesticidal protein according to claim 14 which is capable of killing pests selected from insects, fungi, bacteria, nematodes, mites, ticks, protozoan pathogens, and animal parasites.

17. A pesticidal protein according to claim 16, wherein said insects are selected from the orders Coleoptera, Diptera, Hymenoptera, Lepidoptera, Homoptera, Hemiptera, Orthroptera, Thysanoptera, Dermaptera, Isoptera, Mallophaga, Anoplura, Siphonaptera, or Trichoptera.

18. A pesticidal protein according to claim 17, wherein said coleopteran species is a *Diabrotica*.

19. A pesticidal protein according to claim 18, wherein said *Diabrotica* is *Diabrotica virgifera virgifera* or *Diabrotica longicornis barberi.*

20. A pesticidal protein according to claim 17, wherein said lepidopteran species is an *Agrotis.*

21. A pesticidal protein according to claim 20, wherein said *Agrotis* is *Agrotis ipsilon.*

22. A pesticidal protein according to claim 14, wherein said *Bacillus* is *Bacillus cereus.*

23. A pesticidal protein according to claim 22, wherein said *Bacillus cereus* is *Bacillus cereus* having accession no. B-21058.

24. A pesticidal protein according to claim 14, wherein said *Bacillus* is *Bacillus thuringensis.*

25. A pesticidal protein according to claim 24, wherein said *Bacillus thuringensis* is *Bacillus thuringensis* selected from accession numbers NRRL B-21060, NRRL B-21224, NRRL B-21225, NRRL B-21226 and NRRL B-21227.

26. A pesticidal protein according to claim 14, wherein said protein has a molecular weight of 30 kd or greater.

27. A pesticidal protein according to claim 26, wherein said protein has a molecular weight of about 60 to about 100 kd.

28. A pesticidal protein according to claim 27, wherein said protein has a molecular weight of about 80 kd.

29. A pesticidal protein according to claim 28, wherein said protein has the sequence given in SEQ ID NO:7.

30. A pesticidal protein according to claim 27, wherein said protein has the sequence given in SEQ ID NO:5.

31. A pesticidal protein according to claim 14, wherein said protein comprises an N-terminal sequence as set forth in SEQ ID NO:10 or SEQ ID NO:11.

32. A nucleotide sequence which encodes the protein of claim 14.

33. A nucleotide sequence which encodes the protein of claim 29.

34. A nucleotide sequence which encodes the protein of claim 30.

35. A nucleotide sequence which encodes the protein of claim 31.

36. A nucleotide sequence according to claim 32, wherein said sequence has been optimized for expression in a plant.

37. A nucleotide sequence according to claim 36, wherein said plant is selected from maize, soybean, cotton, wheat, sunflower, tomato, potato, and oilseed rape.

38. A nucleotide sequence according to claim 33, wherein said sequence has been optimized for expression in a plant.

39. A nucleotide sequence according to claim 38, wherein said plant is selected from maize, soybean, cotton, wheat, sunflower, tomato, potato, and oilseed rape.

40. A nucleotide sequence according to claim 34, wherein said sequence has been optimized for expression in a microorganism.

41. A nucleotide sequence according to claim 40, wherein said sequence is set forth in SEQ ID NO:18.

42. A nucleotide sequence according to claim 35, wherein said sequence has been optimized for expression in a microorganism.

43. A nucleotide sequence according to claim 42, wherein said sequence is set forth in SEQ ID NO:17.

44. A nucleotide sequence according to claim 32, wherein said sequence has been optimized for expression in a microorganism.

45. A nucleotide sequence according to claim 44, wherein said microorganism is selected from *Bacillus, Pseudomonas, Saccharomyces, Clavibacter, Erwinia, Serratia, Klebsiella,* *Xanthomonas, Streptomyces, Agrobacterium,* insect pathogenic viruses, fungi, protozoans and nematodes.

46. A plant which has been stably transformed with the nucleotide sequence of any one of claims 32 to 39.

47. A plant according to claim 46, wherein said plant is a maize plant.

48. A nucleotide sequence encoding a pesticidal protein according to claim 14, wherein said sequence is contained in *E. coli* clone P5-4 having accession no. B-21059.

49. A nucleotide sequence encoding a pesticidal protein according to claim 14, wherein said sequence is contained in *E. coli* clone P3-12 having accession no. B-21061.

50. A nucleotide sequence encoding a pesticidal protein according to claim 14, wherein said sequence is contained in *E. coli* clone pCIB 6022 having accession no. NRRL B-21222.

51. A nucleotide sequence according to claim 50 wherein said sequence is given as VIP-1 in SEQ ID NO:1.

52. A method for producing the pesticidal protein of claim 14 **characterized by** the following steps:
a) growing *Bacillus* cells in a culture medium;
b) removing the cells during vegetative growth; and
c) isolating and purifying the pesticidal protein from the liquid culture medium.

## Patentansprüche

1. Bacillus-Stamm, der aus der Gruppe ausgewählt ist, die aus den Stämmen mit den Eingangsnummern B-21058, B-21060, NRRL B-21225, NRRL B-21226, NRRL B-21227, NRRL B-21228, NRRL B-21229 und NRRL B-21230 besteht, worin der Stamm ein pestizides Protein während des vegetativen Wachstums erzeugt, das aus Flüssigkulturmedien isolierbar ist, worin das Protein durch eine Nukleotidsequenz codiert wird, die mit einer Nukleotidsequenz von SEQ ID NO: 1, 4, 6, 9, 17 oder 18 hybridisiert, oder worin das Protein mit Antikörpern kreuzreagiert, die gegen die Proteine von SEQ ID NO: 2, 3, 5, 7, 8 oder 10-16 erzeugt wurden.

2. Bacillus-Stamm gemäss Anspruch 1, worin das Protein Schädlinge töten kann, die aus Insekten, Pilzen, Bakterien, Nematoden, Milben, Zecken, protozoären Pathogenen und tierischen Parasiten ausgewählt sind.

3. Bacillus-Stamm gemäss Anspruch 2, worin das Protein Insekten töten kann, die aus den Ordnungen Coleoptera, Diptera, Hymenoptera, Lepidoptera, Homoptera, Hemiptera, Orthroptera, Thysanoptera, Dermaptera, Isoptera, Mallophaga, Anoplura, Siphonaptera oder Trichoptera ausgewählt sind.

4. Bacillus-Stamm gemäss Anspruch 3, worin die Coleoptera-Art eine Diabrotica ist.

5. Bacillus-Stamm gemäss Anspruch 4, worin die Diabrotica Diabrotica virgifera virgifera oder Diabrotica longicornis barberi ist.

6. Bacillus-Stamm gemäss Anspruch 3, worin die Lepidoptera-Art ein Agrotis ist.

7. Bacillus-Stamm gemäss Anspruch 6, worin das Agrotis Agrotis ipsilon ist.

8. Bacillus-Stamm gemäss Anspruch 1, worin das Protein ein Molekulargewicht von 30 kD oder grösser hat.

9. Bacillus-Stamm gemäss Anspruch 8, worin das Protein ein Molekulargewicht von ca. 60 bis ca. 100 kD hat.

10. Bacillus-Stamm gemäss Anspruch 9, worin das Protein ein Molekulargewicht von ca. 80 kD hat.

11. Bacillus-Stamm gemäss Anspruch 10, worin das Protein die in SEQ ID NO: 7 angegebene Sequenz hat.

12. Bacillus-Stamm gemäss Anspruch 9, worin das Protein ein Molekulargewicht von ca. 100 kD hat.

13. Bacillus-Stamm gemäss Anspruch 12, worin das Protein die in SEQ ID NO: 5 angegebene Sequenz hat.

14. Pestizides Protein, das während der vegetativen Wachstumsphase von Bacillus spp. isolierbar ist, oder aktive Fragmente davon, worin das Protein aus Flüssigkulturmedien isolierbar ist, worin das Protein durch eine Nukleotidsequenz codiert wird, die mit einer Nukleotidsequenz von SEQ ID NO: 1, 4, 6, 9, 17 oder 18 hybridisiert, oder worin das Protein mit Antikörpern kreuzreagiert, die gegen die Proteine von SEQ ID NO: 2, 3, 5, 7, 8 oder 10-16 erzeugt wurden.

15. Pestizides Protein gemäss Anspruch 14, das ein multimeres Protein ist.

16. Pestizides Protein gemäss Anspruch 14, das Schädlinge töten kann, die aus Insekten, Pilzen, Bakterien, Nematoden, Milben, Zecken, protozoären Pathogenen und tierischen Parasiten ausgewählt sind.

17. Pestizides Protein gemäss Anspruch 16, worin die Insekten aus den Ordnungen Coleoptera, Diptera, Hymenoptera, Lepidoptera, Homoptera, Hemiptera, Orthroptera, Thysanoptera, Dermaptera, Isoptera, Mallophaga, Anoplura, Siphonaptera oder Trichoptera ausgewählt sind.

18. Pestizides Protein gemäss Anspruch 17, worin die Coleoptera-Art eine Diabrotica ist.

19. Pestizides Protein gemäss Anspruch 18, worin die Diabrotica Diabrotica virgifera virgifera oder Diabrotica longicornis barberi ist.

20. Pestizides Protein gemäss Anspruch 17, worin die Lepidoptera-Art ein Agrotis ist.

21. Pestizides Protein gemäss Anspruch 20, worin das Agrotis Agrotis ipsilon ist.

22. Pestizides Protein gemäss Anspruch 14, worin der Bacillus Bacillus cereus ist.

23. Pestizides Protein gemäss Anspruch 22, worin der Bacillus cereus Bacillus cereus mit der Eingangsnummer B-21058 ist.

24. Pestizides Protein gemäss Anspruch 14, worin der Bacillus Bacillus thuringensis ist.

25. Pestizides Protein gemäss Anspruch 24, worin der Bacillus thuringensis ein Bacillus thuringensis ist, der aus den Eingangsnummern NRRL B-21060, NRRL B-21224, NRRL B-21225, NRRL B-21226 und NRRL B-2.1227 ausgewählt ist.

26. Pestizides Protein gemäss Anspruch 14, worin das Protein ein Molekulargewicht von 30 kD oder grösser hat.

27. Pestizides Protein gemäss Anspruch 26, worin das Protein ein Molekulargewicht von ca. 60 bis ca. 100 kD hat.

28. Pestizides Protein gemäss Anspruch 27, worin das Protein ein Molekulargewicht von ca. 80 kD hat.

29. Pestizides Protein gemäss Anspruch 28, worin das Protein die in SEQ ID NO: 7 angegebene Sequenz hat.

30. Pestizides Protein gemäss Anspruch 27, worin das Protein die in SEQ ID NO: 5 angegebene Sequenz hat.

31. Pestizides Protein gemäss Anspruch 14, worin das Protein eine wie in SEQ ID NO: 10 oder SEQ ID NO: 11 angegebene N-terminale Sequenz umfasst.

32. Nukleotidsequenz, die das Protein gemäss Anspruch 14 codiert.

33. Nukleotidsequenz, die das Protein gemäss Anspruch 29 codiert.

34. Nukleotidsequenz, die das Protein gemäss Anspruch 30 codiert.

35. Nukleotidsequenz, die das Protein gemäss Anspruch 31 codiert.

36. Nukleotidsequenz gemäss Anspruch 32, worin die Sequenz zur Expression in einer Pflanze optimiert wurde.

37. Nukleotidsequenz gemäss Anspruch 36, worin die Pflanze aus Mais, Sojabohne, Baumwolle, Weizen, Sonnenblume, Tomate, Kartoffel und Ölraps ausgewählt ist.

38. Nukleotidsequenz gemäss Anspruch 33, worin die Sequenz zur Expression einer Pflanze optimiert wurde.

39. Nukleotidsequenz gemäss Anspruch 38, worin die Pflanze aus Mais, Sojabohne, Baumwolle, Weizen, Sonnenblume, Tomate, Kartoffel und Ölraps ausgewählt ist.

40. Nukleotidsequenz gemäss Anspruch 34, worin die Sequenz zur Expression in einem Mikroorganismus optimiert wurde.

41. Nukleotidsequenz gemäss Anspruch 40, worin die Sequenz in SEQ ID NO: 18 dargestellt ist.

42. Nukleotidsequenz gemäss Anspruch 35, worin die Sequenz zur Expression in einem Mikroorganismus optimiert wurde.

43. Nukleotidsequenz gemäss Anspruch 42, worin die Sequenz in SEQ ID NO: 17 dargestellt ist.

44. Nukleotidsequenz gemäss Anspruch 32, worin die Sequenz zur Expression in einem Mikroorganismus optimiert wurde.

45. Nukleotidsequenz gemäss Anspruch 44, worin der Mikroorganismus aus Bacillus, Pseudomonas, Saccharomyces, Clavibacter, Erwinia, Serratia, Klebsiella, Xanthomonas, Streptomyces, Agrobacterium, insektenpathogenen Viren, Pilzen, Protozoen und Nematoden ausgewählt ist.

46. Pflanze, die stabil mit der Nukleotidsequenz gemäss einem der Ansprüche 32 bis 39 transformiert wurde.

47. Pflanze gemäss Anspruch 46, worin die Pflanze eine Maispflanze ist.

48. Nukleotidsequenz, die ein pestizides Protein gemäss Anspruch 14 codiert, worin die Sequenz in E. coli-Klon P5-4 mit der Eingangsnummer B-21059 enthalten ist.

49. Nukleotidsequenz, die ein pestizides Protein gemäss Anspruch 14 codiert, worin die Sequenz in E. coli-Klon P3-12 mit der Eingangsnummer B-21061 enthalten ist.

50. Nukleotidsequenz, die ein pestizides Protein gemäss Anspruch 14 codiert, worin die Sequenz in E. coli-Klon pCIB 6022 mit der Eingangsnummer NRRL B-21222 enthalten ist.

51. Nukleotidsequenz gemäss Anspruch 50, worin die Sequenz als VIP-1 in SEQ ID NO: 1 angegeben ist.

52. Verfahren zur Erzeugung des pestiziden Proteins gemäss Anspruch 14, **gekennzeichnet durch** die folgenden Schritte:
(a) Züchten von Bacillus-Zellen in einem Kulturmedium;
(b) Entfernen der Zellen während des vegetativen Wachstums; und
(c) Isolieren und Reinigen des pestiziden Proteins aus dem Flüssigkulturmedium.

## Revendications

1. Souche de *Bacillus* choisie dans le groupe constitué par les souches ayant les numéros d'accès B-21058, B21060, NRRL B-21225, NRRL B-21226, NRRL B-21227, NRRL B-21228, NRRL B-21229 et NRRL B-21230, dans laquelle ladite souche produit une protéine pesticide pendant la croissance végétative qui peut être isolée à partir de milieux de culture liquides, dans laquelle ladite protéine est codée par une séquence nucléotidique qui s'hybride avec une séquence nucléotidique de SEQ ID n° 1, 4, 6, 9, 17 ou 18, ou dans laquelle ladite protéine inter-réagit avec des anticorps dirigés contre les protéines de SEQ ID n° 2, 3, 5, 7, 8, ou 10 à 16.

2. Souche de *Bacillus* selon la revendication 1, dans laquelle ladite protéine est capable de détruire des agents nuisibles choisis parmi les insectes, les champignons, les bactéries, les nématodes, les acariens, les tiques, les pathogènes protozoaires et les parasites des animaux.

3. Souche de *Bacillus* selon la revendication 2, dans laquelle ladite protéine est capable de détruire des insectes choisi parmi les ordres Coleoptera, Diptera, Hymenophtera, Lepidoptera, Homoptera, Hemiphtera, Orthroptera, Thysanoptera, Dermaptera, Isoptera, Mallophaga, Anoplura, Siphonaptera, ou Trichoptera.

4. Souche de *Bacillus* selon la revendication 3, dans laquelle ladite espèce de coléoptères est un *Diabrotica.*

5. Souche de *Bacillus* selon la revendication 4, dans laquelle ledit *Diabrotica* est *Diabrotica virgifera virgifera* ou *Diabrotica Iongicornis barberi*.

6. Souche de *Bacillus* selon la revendication 3, dans laquelle ladite espèce de lépidoptères est un *Agrotis.*

7. Souche de *Bacillus* selon la revendication 6, dans laquelle ledit *Agrotis* est *Agrotis ipsilon.*

8. Souche de *Bacillus* selon la revendication 1, dans laquelle ladite protéine a un poids moléculaire de 30 kd ou plus.

9. Souche de *Bacillus* selon la revendication 1, dans laquelle ladite protéine a un poids moléculaire allant d'environ 60 kd à 100 kd.

10. Souche de *Bacillus* selon la revendication 9, dans laquelle ladite protéine a un poids moléculaire d'environ 80 kd.

11. Souche de *Bacillus* selon la revendication 10, dans laquelle ladite protéine a la séquence donnée dans SEQ ID n° 7.

12. Souche de *Bacillus* selon la revendication 9, dans laquelle ladite protéine a un poids moléculaire d'environ 100 kd.

13. Souche de *Bacillus* selon la revendication 12, dans laquelle ladite protéine a la séquence donnée dans SEQ ID n° 5.

14. Protéine pesticide qui peut être isolée pendant la phase de croissance végétative de *Bacillus* spp. ou fragments actifs de celle-ci, dans laquelle ladite protéine peut être isolée à partir de milieux de culture liquides, et dans laquelle ladite protéine est codée par une séquence nucléotidique qui s'hybride avec une séquence nucléotidique de SEQ ID n° 1, 4, 6, 9, 17 ou 18, ou dans laquelle ladite protéine inter-réagit avec des anticorps dirigés contre les protéines de SEQ ID n° 2, 3, 5, 7, 8, ou 10 à 16.

15. Protéine pesticide selon la revendication 14 qui est une protéine multimère.

16. Protéine pesticide selon la revendication 14 qui est capable de détruire des agents nuisibles choisis parmi les insectes, les champignons, les bactéries, les nématodes, les acariens, les tiques, les pathogènes protozoaires et les parasites des animaux.

17. Protéine pesticide selon la revendication 16 dans laquelle lesdits insectes sont choisis parmi les ordres Coleoptera, Diptera, Hymenophtera, Lepidoptera, Homoptera, Hemiphtera, Orthroptera, Thysanoptera, Dermaptera, Isoptera, Mallophaga, Anoplura, Siphonaptera, ou Trichoptera.

18. Protéine pesticide selon la revendication 17, dans laquelle ladite espèce de coléoptères est un Diabrotica.

19. Protéine pesticide selon la revendication 18, dans laquelle ledit *Diabrotica* est *Diabrotica virgifera virgifera* ou *Diabrotica longicornis barberi*.

20. Protéine pesticide selon la revendication 17, dans laquelle ladite espèce de lépidoptères est un Agrotis.

21. Protéine pesticide selon la revendication 20, dans laquelle ledit *Agrotis* est *Agrotis ipsilon.*

22. Protéine pesticide selon la revendication 14, dans laquelle ledit *Bacillus* est *Bacillus cereus.*

23. Protéine pesticide selon la revendication 22, dans laquelle ledit *Bacillus cereus* est le *Bacillus cereus* ayant le numéro d'accès n° B-21058.

24. Protéine pesticide selon la revendication 14, dans laquelle ledit *Bacillus* est *Bacillus thuringiensis.*

25. Protéine pesticide selon la revendication 24, dans laquelle ledit *Bacillus thuringiensis* est le *Bacillus thuringiensis* choisi parmi les numéros d'accès NRRL B-21060, NRRL B-21224, NRRL B-21225, NRRL B-21226 et NRRL B-21227.

26. Protéine pesticide selon la revendication 14, dans laquelle ladite protéine a un poids moléculaire de 30 kd ou plus.

27. Protéine pesticide selon la revendication 26, dans laquelle ladite protéine a un poids moléculaire allant d'environ 60 kd à environ 100 kd.

28. Protéine pesticide selon la revendication 27, dans laquelle ladite protéine a un poids moléculaire d'environ 80 kd.

29. Protéine pesticide selon la revendication 28, dans laquelle ladite protéine a la séquence donnée dans SEQ ID n° 7.

30. Protéine pesticide selon la revendication 27, dans laquelle ladite protéine a la séquence donnée dans SEQ ID n° 5.

31. Protéine pesticide selon la revendication 14, dans laquelle ladite protéine comprend une séquence N terminale telle que décrite dans SEQ ID n° 10 ou SEQ ID n° 11.

32. Séquence nucléotidique qui code pour la protéine selon la revendication 14.

33. Séquence nucléotidique qui code pour la protéine selon la revendication 29.

34. Séquence nucléotidique qui code pour la protéine selon la revendication 30.

35. Séquence nucléotidique qui code pour la protéine selon la revendication 31.

36. Séquence nucléotidique selon la revendication 32, dans laquelle ladite séquence a été optimisée pour être exprimée dans une plante.

37. Séquence nucléotidique selon la revendication 36, dans laquelle ladite plante est choisi parmi le maïs, le soja, le coton, le blé, le tournesol, la tomate, la pomme de terre, et la navette.

38. Séquence nucléotidique selon la revendication 33, dans laquelle ladite séquence a été optimisée pour être exprimée dans une plante.

39. Séquence nucléotidique selon la revendication 38, dans laquelle ladite plante est choisi parmi le mais, le soja, le coton, le blé, le tournesol, la tomate, la pomme de terre, et la navette.

40. Séquence nucléotidique selon la revendication 34, dans laquelle ladite séquence a été optimisée pour être exprimée dans un microorganisme.

41. Séquence nucléotidique selon la revendication 40, dans laquelle ladite séquence est décrite dans SEQ ID n° 18.

42. Séquence nucléotidique selon la revendication 35, dans laquelle ladite séquence a été optimisée pour être exprimée dans un microorganisme.

43. Séquence nucléotidique selon la revendication 42, dans laquelle ladite séquence est décrite dans SEQ ID n° 17.

44. Séquence nucléotidique selon la revendication 32, dans laquelle ladite séquence a été optimisée pour être exprimée dans un microorganisme.

45. Séquence nucléotidique selon la revendication 44, dans laquelle ledit microorganisme est choisi parmi *Bacillus, Pseudomonas, Saccharomyces, Clavibacter, Erwinia, Serratia, Klebsiella, Xanthomonas*, *Streptomyces, Agrobacterium,* les virus pathogènes des insectes, les champignons, les protozoaires et les nématodes.

46. Plante qui a été transformée de façon stable avec la séquence nucléotidique selon l'une quelconque des revendications 32 à 39.

47. Plante selon la revendication 46, dans laquelle ladite plante est une plante de maïs.

48. Séquence nucléotidique codant pour une protéine pesticide selon la revendication 14, dans laquelle ladite séquence est contenue dans le clone de *E.coli* P5-4 ayant le numéro d'accès n° B-21059.

49. Séquence nucléotidique codant pour une protéine pesticide selon la revendication 14, dans laquelle ladite séquence est contenue dans le clone de E.coli P3-12 ayant le numéro d'accès n° B-21061.

50. Séquence nucléotidique codant pour une protéine pesticide selon la revendication 14, dans laquelle ladite séquence est contenue dans le clone de *E.coli* pCIB 6022 ayant le numéro d'accès n° NRRL B-21222.

51. Séquence nucléotidique selon la revendication 50 dans laquelle ladite séquence est donnée comme VIP-1 dans SEQ ID n° 1.

52. Procédé de production de la protéine pesticide selon la revendication 14 **caractérisé par** les étapes suivantes :
a) faire croître des cellules de *Bacillus* dans un milieu de culture ;
b) retirer les cellules pendant la croissance végétative ; et
c) isoler et purifier la protéine pesticide à partir du milieu de culture liquide.
